# EUROPÄISCHE PATENTANMELDUNG

(11) **EP 2 048 126 A1**
(43) Veröffentlichungstag der Anmeldung: **15.04.2009**
(21) Anmeldenummer: 07075893.3
(22) Anmeldetag: 11.10.2007
(51) Int. Cl.: C07C 39/14, C07C 39/23, C07C 39/42, A61K 31/05, A61K 31/055, A61P 5/30, A61P 15/00

(54) **Benzocycloheptanderivate als selektiv wirksame Estrogene**

(71) Anmelder: Bayer Schering Pharma AG, 13353 Berlin (DE)
(72) Erfinder: Wyrwa, Ralf, Dr., 07751 Rothenstein (DE); Peters, Olaf, Dr., 99891 Tabarz (DE); Bohlmann, Rolf, Dr., 14055 Berlin (DE); Droescher, Peter, Dr., 14195 Berlin (DE); Prelle, Katja, Dr., 13465 Berlin (DE); Fritzemeier, Karl-Heinrich, Dr., 13505 Berlin (DE); Muhn, Hans-Peter, Dr., 13465 Berlin (DE)

(57) **Zusammenfassung**

Die vorliegende Erfindung beschreibt nicht-steroidale Verbindungen der allgemeinen Formel (I) 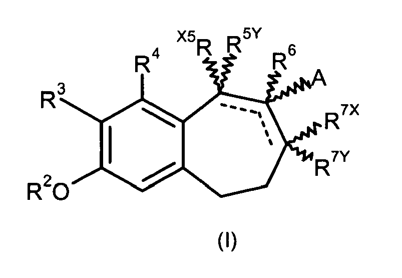 worin
A 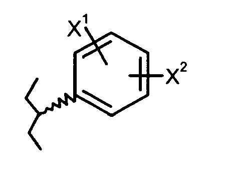 bedeutet worin
X¹ ein oder mehrere Gruppen am Phenylring bedeutet und unabhängig von einander ein Halogen, OH, (C₁-C₄)Alkyl-, (C₁-C₄)Alkyl-O-, (C₃-C₆)Cyclo-alkyl-O, (C₁-C₁₄)Acyl-O-, (C₁-C₄)Alkenyl-, (C₁-C₄)Alkinyl-, Perfluor(C₁-C₄)Alkylrest, -CHO oder CN darstellt, und
X² ein oder mehrere Gruppen am Phenylring bedeutet und unabhängig von einander ein H, Halogen, OH, (C₁-C₄)Alkyl-, (C₁-C₄)Alkyl-O-, (C₁-C₄)Alkenyl-, (C₁-C₄)Alkinyl-, Perfluor(C₁-C₄)Alkylrest, -CHO oder CN darstellt, als Estrogene.

## Beschreibung

### Feld der Erfindung

Die vorliegende Erfindung bezieht sich auf neue Verbindungen der allgemeinen Formel (I) 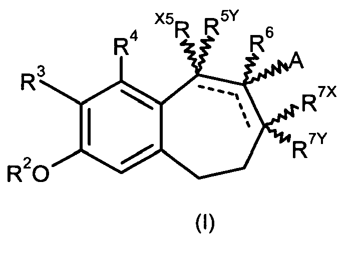 worin
A 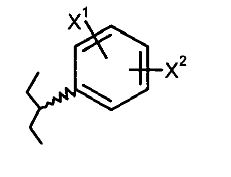 bedeutet worin
- X¹: ein oder mehrere Gruppen am Phenylring bedeutet und unabhängig von einander ein Halogen, OH, (C₁-C₄)Alkyl-, (C₁-C₄)Alkyl-O-, (C₃-C₆)Cyclo-alkyl-O, (C₁-C₁₄)Acyl-O-, (C₁-C₄)Alkenyl-, (C₁-C₄)Alkinyl-, Perfluor(C₁-C₄)Alkylrest, -CHO oder CN darstellt, und
- X²,: ein oder mehrere Gruppen am Phenylring bedeutet und unabhängig von einander ein H, Halogen, OH, (C₁-C₄)Alkyl-, (C₁-C₄)Alkyl-O-, (C₁-C₄)Alkenyl-, (C₁-C₄)Alkinyl-, Perfluor(C₁-C₄)Alkylrest, -CHO oder CN darstellt;
- R²: ein H-Atom, ein (C₁-C₄)Alkylrest, ein (C₃-C₆)Cycloalkyl-rest oder ein (C₁-C₁₄)Acylrest ist;
- R³: ein H- oder ein F-Atom ist;
- R⁴: ein H-Atom, ein (C₁-C₄)Alkyl-, (C₁-C₄)Alkenyl-, (C₁-C₄)Alkinyl-, Perfluor(C₁-C₄)alkyl-, Silyl(C₁-C₄)alkylrest, ein Halogenatom oder ein Nitrilrest ist;
- R^{5X}und R^{5Y}: unabhängig von einander ein H-Atom, einen (C₁-C₄)Alkyl-, (C₁-C₄)Alkenyl-, (C₁-C₄)Alkinyl-, Perfluor(C₁-C₄)alkyl-, Perfluor(C₁-C₄)alkenyl-, (C₁-C₄)Alkyl-O-, Aryl-, Heteroaryl- oder eine CN-Gruppe bedeuten, oder
- R^{5X}und R^{5Y}: gemeinsam ein O-, ein S-Atom, ein =CHZ worin Z ein H-Atom, ein (C₁-C₄)Alkyl-, (C₁-C₄)Alkenylrest bedeutet, oder
- R^{5X}und R^{5Y}: gemeinsam ein -(C₂-C₄)Alkandiyl-, (C₂-C₆)Alkendiylrest oder -OCH₂- sind, oder
- R^{5Y}und R⁶: gemeinsam eine Bindung, ein -(C₁-C₄)Alkandiyl- oder einen (C₂-C₆)Alkendiylrest darstellen, und R^{5X} hat die oben gegebenen Bedeutung;
- R⁶: ein H-Atom, ein (C₁-C₄)Alkyl-, ein (C₁-C₄)Alkenyl-, (C₁-C₄)Alkinyl-, ein Perfluor(C₁-C₄)alkyl- oder ein Perfluor(C₁-C₄)alkenylrest bedeutet, oder
- R⁶und R^{5Y}: gemeinsam eine Bindung, ein -(C₁-C₄)Alkandiyl- oder einen (C₂-C₆)Alkendiylrest darstellen, und R^{5X} hat die oben gegebenen Bedeutung oder
- R⁶und R^{7Y}: gemeinsam eine Bindung, ein -(C₁-C₄)Alkandiyl- oder einen (C₂-C₆)Alkendiylrest darstellen, und R^{7X} hat die unter gegebenen Bedeutung;
- R^{7X}und R^{7Y}: unabhängig von einander ein H-Atom, ein (C₁-C₄)-Alkyl-, ein (C₁-C₄)Alkenylrest, ein (C₁-C₄)Alkinyl- darstellen, oder
- R^{7X}und R^{7Y}: gemeinsam ein -(C₂-C₄)Alkandiyl-, oder (C₂-C₆)Alkendiylrest darstellen, oder
- R^{7Y}und R⁶: gemeinsam eine Bindung, ein -(C₁-C₄)Alkandiyl- oder einen (C₂-C₆)Alkendiylrest darstellen, und R^{7X} hat die oben gegebenen Bedeutung, als Estrogene.

Die vorliegende Erfindung bezieht sich weiter auf die neuen nicht-steroidalen Verbindungen der allgemeinen Formel (I) als pharmazeutische Wirkstoffe, deren Herstellung, ihre therapeutische Anwendung und pharmazeutischen Darreichungsformen, die die neuen Verbindungen enthalten.

Die in der vorliegenden Erfindung enthaltenen neuen nicht-steroidalen Estrogenrezeptormodulatoren der allgemeinen Formel (I) sind für die Behandlung von Estrogendefizienz bedingten Erkrankungen (altersbedingt, chirurgisch- oder medikamentös bedingt), zur Prävention und Therapie entzündlicher Erkrankungen, zur Verbesserung der Fertilität und zur Therapie von Fertilitätsstörungen der Frau sowie als Komponente neuartiger Empfängnisverhütungsmittel geeignet.

### Stand der Technik

### Behandlung von Estrogendefizienz-bedingten Erkrankungen

Etablierte Estrogentherapien zur Behandlung von Hormondefizienz-bedingten Beschwerden und die protektive Wirkung von Estrogenen auf Knochen, Gehirn, Gefäß und andere Organsysteme sind als Stand der Technik bekannt.

Die Effizienz von Estrogenen zur Behandlung von Hormondefizienz-bedingten Symptomen wie Hitzewallungen, Atrophie von Estrogenzielorganen und Inkontinenz, sowie die erfolgreiche Anwendung von Estrogen-Therapien zur Verhinderung von Knochenmasseverlust bei peri- und postmenopausalen Frauen ist gut belegt und allgemein akzeptiert (Grady D et al.1992, Ann Intern Med 117: 1016-1037). Ebenso ist gut dokumentiert, daß die Estrogenersatztherapie bei postmenopausalen Frauen oder bei Frauen mit anders bedingter ovarieller Dysfunktion das Risiko von Herzkreislauferkrankungen gegenüber nicht estrogenbehandelten Frauen reduziert (Grady et al., loc. cit.).

Neuere Untersuchungen belegen zudem eine protektive Wirkung von Estrogenen gegen neurodegenerative Erkrankungen, wie z.B. Alzheimersche Krankheit (Henderson 1997, Neurology 48 (Suppl 7): S27-S35; Birge 1997, Neurology 48 (Suppl 7): S36-S41; B.R. Bhavnani, Journal of Steroid Biochemistry & Molecular Biology 85 (2003) 473-482), eine schützende Wirkung auf Gehirnfunktionen, wie Gedächtnisleistung und Lernfähigkeit (McEwen et al. 1997, Neurology 48 (Suppl 7): S8-S15; Sherwin 1997, Neurology 48 (Suppl 7): S21-S26), sowie gegen Hormondefizienz-bedingte Stimmungsschwankungen (Halbreich 1997, Neurology 48 (Suppl 7): S16-S20).

Weiterhin hat sich Estrogenersatztherapie als effektiv hinsichtlich der Reduktion der Inzidenz von Kolonkarzinom erwiesen (Calle EF et al., 1995, J Natl Cancer Inst 87: 517-523).

In der herkömmlichen Estrogen- oder Hormonersatztherapie (Hormone Replacement Therapy = HRT) werden natürliche Estrogene wie Estradiol und konjugierte Estrogene aus Pferdeurin entweder allein oder in Kombination mit einem Gestagen eingesetzt. Anstelle der natürlichen Estrogene können auch durch Veresterung erhaltene Derivate, wie z.B. das 17β-Estradiol-Valerat, eingesetzt werden.
Wegen der stimulierenden Wirkung der verwendeten Estrogene auf das Endometrium, die zu einer Erhöhung des Endometriumkarzinomrisikos führt (Harlap S 1992, Am J Obstet Gynecol 166: 1986-1992), werden in der Hormonersatztherapie vorzugsweise Estrogen/Gestagen-Kombinationspräparate eingesetzt. Die gestagene Komponente in der Estrogen/Gestagen-Kombination vermeidet eine Hypertrophie des Endometriums, allerdings ist mit der gestagenhaltigen Kombination auch das Auftreten unerwünschter Zwischenblutungen verknüpft.

Eine neuere Alternative zu den Estrogen/Gestagen-Kombinationspräparaten stellen selektive Estrogene dar. Bisher werden unter selektiven Estrogenen solche Verbindungen verstanden, die estrogenartig auf Gehirn, Knochen und Gefäßsystem, aufgrund ihrer antiuterotrophen (d.h. antiestrogenen) Partialwirkung aber nicht proliferativ auf das Endometrium wirken.

Eine Klasse von Substanzen, die das gewünschte Profil eines selektiven Estrogens teilweise erfüllen sind die sogenannten 'Selective Estrogen Receptor Modulators' (SERM) (R.F. Kauffman, H.U. Bryant 1995, DNAP 8 (9): 531-539). Es handelt sich hierbei um Partialagonisten des Estrogenrezeptorsubtyps ERα. Dieser Typ von Substanzen ist allerdings ineffektiv hinsichtlich der Therapie akuter postmenopausaler Beschwerden, wie z.B. Hitzewallungen. Als Beispiel für ein SERM sei das kürzlich für die Indikation Osteoporose eingeführte Raloxifen genannt.

Auch Estrogenrezeptormodulatoren mit Präferenz für ERbeta, insbesondere ERbetaselektive Agonisten, können günstige Wirkung auf Gehirnfunktionen, Blase, Darm und das kardiovaskuläre System ausüben, ohne im gleichen Dosisbereich hepatische Estrogenwirkung oder stimulierende Wirkung auf Endometrium und Brust auszuüben. ERbeta Agonisten stellen daher eine neue Option für eine selektive Estrogentherapie und zur Behandlung von Hitzewallungen und Stimmungschwankungen dar. Das Auftreten von Hitzewallungen geht vermutlich auf eine Instabilität des hypothalamischen 'thermoregulatory set point', verursacht durch den Estrogenabfall bei Eintreten der Menopause, zurück (Stearns V, Ullmer L, Loepez JF, Smith Y, Isaacs C, Hayes DF (2002) Hot flushes. The Lancet 360: 1851-1861).
ER Agonisten modulieren Komponeneten des serotonergen Systems, wobei der Neurotransmitter Serotonin eine wichtige Rolle bei der Thermoregulation spielt. Der modulatorische Effekt von ER Agonisten auf das Serotoninrezeptorsystem und den Serotonintransporter wird als essentiell für die Stabilisierung des ',thermoregulatory set point' angesehen. ERbeta wird in Arealen des Hypothalamus mit den Komponenten des serotonergen Systems ko-exprimiert, so dass vermutet werden kann, dass die modulierende Wirkung von ER Agonisten durch ERbeta vermittelt wird. Aktivierung von ERbeta könnte ausreichend sein, um den 'thermoregulatory set point' zu stabilisieren.

**Estrogenrezeptormodulatoren, insbesondere** ERbeta Agonisten zur Prävention **und Therapie entzündlicher Erkrankungen**
Basierend auf neueren Untersuchungen ist davon auszugehen, dass ERbeta eine wichtige regulatorische Funktion bei entzündlichen und Erkrankungen des Immunsystems spielt. Dies gilt insbesondere für Autoimmunerkrankungen, wie z.B. Rheumatoide Arthritis, Multiple Sklerose, Lupus, Morbus Crohn und weitere entzündliche Erkrankungen des Darms und der Haut, wie z.B. Psoriasis. Weiterhin ist die ERβ Modulation relevant bei der Behandlung von Endometriose. Aufgrund von Nachweisen in präklinischen Modellen für entzündliche Erkrankungen des Menschen ist davon auszugehen, dass Estrogenrezeptormodulatoren, insbesondere ER Agonisten mit ERbeta-Präferenz zur Prävention und Behandlung entzündlicher Erkrankungen eingesetzt werden können (Heather, H.A.; Mol Endocrinol. 2007 Jan; 21(1):1-13).

### Estrogenrezeptormodulatoren, insbesondere ERbeta Agonisten, zur Behandlung von Fertilitätsstörungen der Frau

Für die Behandlung von Fertilitätsstörungen der Frau, häufig verursacht durch chirurgisch, medikamentös oder anders bedingte ovarielle Dysfunktion eröffnen sich durch den Einsatz neuer selektiver ER-Modulatoren (Estrogene) ebenfalls neue Therapiemöglichkeiten. Die in vitro Fertilitätsbehandlung ist ein seit mehr als 20 Jahren etabliertes Verfahren. Zahlreiche Methoden zur Behandlung der ovariell bedingten Infertilität mit exogenen Gonadotropinen sind bekannt. Durch Gabe von Gonadotropinen wie FSH (FSH = Follikel-Stimulierendes Hormon) soll eine Stimulierung der Ovarien bewirkt werden, die eine gesunde Follikelreifung ermöglichen soll.

Der Follikel ist die funktionelle Einheit des Ovars und hat zwei Aufgaben: er beherbergt die Oozyte und schafft für diese so die Möglichkeit zum Wachstum und zur Reifung. Die Follikulogenese umfasst die Entwicklung eines ovariellen Follikels vom primordialen Stadium zu einem sich stetig vergrößernden Antralfollikel, der das letzte Stadium vor der Ovulation darstellt. Nur ein optimal entwickelter Antralfollikel kann eine ausgereifte Eizelle durch Ovulation freisetzen.
Patientinnen mit ovariell bedingter Infertilität (PCOS = Syndrom der Polyzystischen Ovarien) leiden an einer gestörten Follikelreifung, welche sowohl mit hormonellen und ovulatorischen Störungen als auch mit unzureichend gereiften Eizellen verbunden ist. Die Anzahl der primären und sekundären Follikel ist hier ungefähr zweimal so hoch wie im normalen Ovar (Hughesden et al., Obstet. Gynecol. Survey 37, 1982, S. 59-77).

Es gibt Hinweise, dass die frühen Entwicklungsstadien der Follikulogenese (das betrifft die Entwicklung vom Primordial- zum Antralfollikel) Gondadotropinunabhängig sind. Es ist nicht eindeutig geklärt, wie groß der Einfluss bekannter parakriner und autokriner Faktoren auf die frühe Follikulogenese ist (Elvin et al., Mol. Cell Endocrinol. 13, 1999, S. 1035-1048; McNatty et al., J. Reprod. Fertil. Suppl.. 54, 1999, S. 3-16).
Gonadotropine wie FSH sind hauptsächlich in den letzten Entwicklungsstadien der Follikulogenese an der Follikelreifung beteiligt, d.h. bei der Entwicklung vom frühen Antralfollikel zu einem reifen ovulationsfähigen Follikel.

Die in vivo und in vitro Infertilität wird vorzugsweise mit Gonadotropinen (FSH und Antiestrogenen) behandelt (White et al., J. Clin. Endocrinol. Metab. 81, 1996, S. 3821-3824). Bei der in vitro Fertilitätsbehandlung werden Oozyten aus präovulatorischen Antralfollikeln entnommen, um sie in vitro zu einer befruchtungsfähigen Eizelle heranreifen zu lassen. Nach der Befruchtung und der präembryonalen Entwicklung werden ein bis drei Embryonen in den Uterus der Frau transferiert.

Die Behandlung mit exogenen Gonadotropinen ist in vielerlei Hinsicht von zahlreichen Risiken und Nebenwirkungen begleitet. Das größte Risiko besteht in einer Überstimulierung der Ovarien, welche in schweren Fällen eine ernsthafte Lebensgefahr darstellen kann (OHSS = Ovarian Hyperstimulation Syndrome). Weitere Nebeneffekte sind die hohen Kosten der in vitro Fertilitätsbehandlung, die von den Paaren getragen werden müssen. Negative Nebenwirkungen wie Gewichtszunahme, Aufgedunsenheit, Übelkeit, Erbrechen und ein bisher unbekanntes Langzeitrisiko, an Krebs zu erkranken, werden der Gonadotropin-Behandlung zugeschrieben.
Eine Methode, um die genannten Nachteile und Risiken zu vermeiden, wird darin gesehen, die Reifung und Stimulierung des follikulären Wachstums bei ovariell bedingter Infertilität in vivo mit einem geeigneten Wirkstoffen, insbesondere selektiven ER-Modulatoren zu veranlassen.

### Estrogenrezeptor beta (ERβ)

Der Estrogenrezeptor-β (ERβ) als zweiter Subtyp des Estrogenrezeptors wurde identifiziert bei Kuiper et al. (1996), Proc. Natl. Acad. Sci. 93:5925-5930 und Mosselman, Dijkema (1996) Febs Letters 392: 49-53; Tremblay et al. (1997), Molecular Endocrinology 11: 353-365.

Das Expressionsmuster von ERβ unterscheidet sich von dem des ERα (Kuiper et al. (1996), Endocrinology 138: 863-870). So überwiegt ERβ gegenüber ERα in der Rattenprostata, während in Rattenuterus ERα gegenüber ERβ überwiegt. Im Gehirn wurden Areale identifiziert, in denen jeweils nur einer der beiden ER-Subtypen exprimiert wird (Shugrue et al. (1996), Steroids 61: 678-681; Li et al. (1997), Neuroendocrinology 66:63-67). ERβ wird u.a. in Arealen exprimiert, denen Bedeutung für kognitive Prozesse und ,Stimmung' zugewiesen wird (Shugrue et al. 1997, J Comparative Neurology 388:507-525).

Weitere Organsysteme mit vergleichsweise hoher ERβ-Expression umfassen den Knochen (Onoe Y et al., 1997, Endocrinology 138:4509-4512), das Gefäßsystem (Register TC, Adams MR 1998, J Steroid Molec Biol 64: 187-191), den Urogenitaltrakt (Kuiper GJM et al. 1997, Endocrinology 138: 863-870), den Gastrointestinaltrakt (Campbell-Thopson 1997, BBRC 240: 478-483), sowie die Testis (Mosselmann S et al. 1996 Febs Lett 392 49-53) einschließlich der Spermatiden (Shugrue et al. 1998, Steroids 63: 498-504). Die Gewebeverteilung legt nahe, daß Estrogene über ERβ Organfunktionen regulieren. Daß ERβ in dieser Hinsicht funktionell ist, ergibt sich auch durch Untersuchungen an ERα- (ERKO) bzw. ERβ-(βERKO)-Knockout-Mäusen: Ovariektomie bewirkt Knochenmasseverlust in ERKO-Mäusen, der durch Estrogensubstitution aufgehoben werden kann (Kimbro et al. 1998, Abstract OR7-4, Endocrine Society Meeting New Orleans). Ebenso hemmt Estradiol in Blutgefäßen weiblicher ERKO-Mäuse die Gefäßmedia- und Glattmuskelzellproliferation (lafrati MD et al. 1997, Nature Medicine 3: 545-548). Diese protektiven Wirkungen von Estradiol erfolgen in der ERKO-Maus vermutlich über ERβ.
Beobachtungen an βERKO-Mäusen liefern einen Hinweis auf eine Funktion von ERβ in Prostata und Blase: bei älteren männlichen Mäusen treten Symptome von Prostata- und Blasenhyperplasie auf (Krege JH et al. 1998, Proc Natl Acad Sci 95: 15677-15682). Außerdem weisen weibliche (Lubahn DB et al. 1993, Proc Natl Acad Sci 90:11162-11166) und männliche ERKO-Mäuse (Hess RA et al. 1997, Nature 390: 509-512) sowie weibliche βERKO-Mäuse (Krege JH, 1998) Fertilitätsstörungen auf. Hierdurch wird die wichtige Funktion von Estrogenen hinsichtlich Aufrechterhaltung von Testis- und Ovarfunktion sowie Fertilität belegt.

Westerlind et al. (1998) beschreiben eine differentielle Wirkung von 16α-Hydroxyestron auf den Knochen einerseits und Reproduktionsorgane der weiblichen Ratte andererseits (J Bone and Mineral Res 13: 1023-1031).
Eigene Untersuchungen ergaben, daß 16α-Hydroxyestron 3-fach besser an den humanen Estrogenrezeptor β (ERβ) als an den humanen Estrogenrezeptor α (ERα) bindet. Der RBA-Wert der Substanz am Rattenprostataestrogenrezeptor ist 5-fach besser als der RBA-Wert der Substanz am Rattenuterusestrogenrezeptor. Die von Westerlind beschriebene Dissoziation der Substanz ist nach eigenen Erkenntnissen auf ihre Präferenz für ERβ im Vergleich zu ERα zurückzuführen.

Eine selektive Estrogenwirkung auf bestimmte Zielorgane könnte aufgrund der unterschiedlichen Gewebe- bzw. Organverteilung der beiden Subtypen des ERs durch subtypspezifische Liganden erreicht werden. Substanzen mit Präferenz für ERβ verglichen mit ERα im in vitro Rezeptorbindungstest wurden von Kuiper et al. beschrieben (1996, Endocrinology 138: 863-870).
Die Rolle von selektiven ERβ Liganden ist weiter erläutert in mehreren Veröffentlichung und Reviews wie zum Beispiel in "Estrogen receptor-β: Recent lessons from in vivo studies" Harris H.A., Molecular Endocrinology, 2006**,** "Benzopyrans are selective Estrogen Receptor-β Agonists with novel activity in models of Benign Prostatic Hyperplasia", Krishnan V. et al., J. Med. Chem. 2006, 49, 6155-6157 und "Estrogen Receptor beta in Health and Disease", Gustafsson, J.A. et al., Biology of Reproduction, 2005, 73, 866-871.

### Estrogenrezeptormodulatoren, insbesondere ER Agonisten oder Estrogene als Komponente oraler Kontrazeptiva

Kontrazeptive Methoden, die auf der Ovulationshemmung durch Applikation mit einer Kombination aus einem Estrogen und einem Gestagen beruhen, sind sehr gut etabliert. ER-Modulatoren sind als estrogene Komponente von Kombinationspräparaten für die Kontrazeption besonders gut geeignet. Zielorgane des Estrogens in Kombinationspräparaten sind insbesondere die Hypophyse, das Ovar und das Endometrium. Diese Organe exprimieren ERα (Kuiper et al. (1996), Endocrinology 138: 863-870).

### Bekannte Benzocycloheptenderivate

WO03/033461 betrifft Zwischenprodukte und ein neues Verfahren zur Herstellung des Benzocycloheptens. Das Verfahren zur Herstellung dessen Zwischenprodukte geht von kostengünstigen Ausgangsmaterialien aus, liefert die Zwischenstufen in hohen Ausbeuten und hoher Reinheit, ohne chromatographische Reinigungsschritte und erlaubt die Herstellung im großen Maßstab.

WO00/03979 stellt weitere SERMs mit Benzocyclohepten-Grundkörper, Verbindungen mit starker antiestrogener Wirksamkeit dar. Es handelt sich hierbei um selektive Estrogene, deren Wirkung gewebeselektiv auftritt. Insbesondere tritt die estrogene Wirkung am Knochen auf. Vorteil dieser Verbindungsklasse ist, dass am Uterus und in der Leber keine oder nur äusserst geringfügige Wirkung auftritt.

Die Verbindungen nach WO00/03979 und WO03/033461 können auch über antiestrogene Wirksamkeit verfügen, die sich beispielsweise im Antiuteruswachstumstest oder in Tumormodellen nachweisen lässt.
Verbindungen mit einem derartigen Wirkprofil werden als Selective Estrogen Receptor Modulators(SERMs) bezeichnet, wie schon vorher beschrieben.

### Offenbarung der Erfindung

Aufgabe der vorliegenden Erfindung ist es, nicht-steroidale Verbindungen mit estrogener Wirkung bereitzustellen.

Erfindungsgemäß gelöst wird die vorstehende Aufgabe durch die Bereitstellung von Verbindungen der allgemeinen Formel (I) 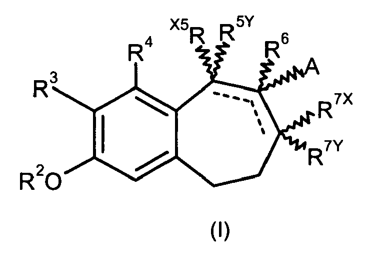 worin
A 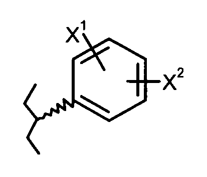 bedeutet worin
- X¹: ein oder mehrere Gruppen am Phenylring bedeutet und unabhängig von einander ein Halogen, OH, (C₁-C₄)Alkyl-, (C₁-C₄)Alkyl-O-, (C₃-C₆)Cyclo-alkyl-O, (C₁-C₁₄)Acyl-O-, (C₁-C₄)Alkenyl-, (C₁-C₄)Alkinyl-, Perfluor(C₁-C₄)Alkylrest, -CHO oder CN darstelt, und
- X²: ein oder mehrere Gruppen am Phenylring bedeutet und unabhängig von einander ein H, Halogen, OH, (C₁-C₄)Alkyl-, (C₁-C₄)Alkyl-O-, (C₁-C₄)Alkenyl-, (C₁-C₄)Alkinyl-, Perfluor(C₁-C₄)Alkylrest, -CHO oder CN darstellt;
- R²: ein H-Atom, ein (C₁-C₄)Alkylrest, ein (C₃-C₆)Cyclo-alkyl-rest oder ein (C₁-C₁₄)Acylrest ist;
- R³: ein H- oder ein F-Atom ist;
- R⁴: ein H-Atom, ein (C₁-C₄)Alkyl-, (C₁-C₄)Alkenyl-, (C₁-C₄)Alkinyl-, Perfluor(C₁-C₄)alkyl-, Silyl(C₁-C₄)alkylrest, ein Halogenatom oder ein Nitrilrest ist;
- R^{5X}und R^{5Y}: unabhängig von einander ein H-Atom, einen (C₁-C₄)Alkyl-, (C₁-C₄)Alkenyl-, (C₁-C₄)Alkinyl-, Perfluor(C₁-C₄)alkyl-, Perfluor(C₁-C₄)alkenyl-, (C₁-C₄)Alkyl-O-, Aryl-, Heteroaryl- oder eine CN-Gruppe bedeuten, oder
- R^{5X}und R^{5Y}: gemeinsam ein O-, ein S-Atom, ein =CHZ worin Z ein H-Atom, ein (C₁-C₄)Alkyl-, (C₁-C₄)Alkenylrest bedeutet, oder
- R^{5X}und R^{5Y}: gemeinsam ein -(C₂-C₄)Alkandiyl-, (C₂-C₆)Alkendiylrest oder -OCH₂- sind, oder
- R^{5Y}und R⁶: gemeinsam eine Bindung, ein -(C₁-C₄)Alkandiyl- oder einen (C₂-C₆)Alkendiylrest darstellen, und R^{5X} hat die oben gegebenen Bedeutung;
- R⁶: ein H-Atom, ein (C₁-C₄)Alkyl-, ein (C₁-C₄)Alkenyl-, (C₁-C₄)Alkinyl-, ein Perfluor(C₁-C₄)alkyl- oder ein Perfluor(C₁-C₄)alkenylrest bedeutet, oder
- R⁶und R^{5Y}: gemeinsam eine Bindung, ein -(C₁-C₄)Alkandiyl- oder einen (C₂-C₆)Alkendiylrest darstellen, und R^{5X} hat die oben gegebenen Bedeutung oder
- R⁶und R^{7Y}: gemeinsam eine Bindung, ein -(C₁-C₄)Alkandiyl- oder einen (C₂-C₆)Alkendiylrest darstellen, und R^{7X} hat die unter gegebenen Bedeutung;
- R^{7X}und R^{7Y}: unabhängig von einander ein H-Atom, ein (C₁-C₄)-Alkyl-, ein (C₁-C₄)Alkenylrest, ein (C₁-C₄)Alkinyl-, oder
- R^{7X}und R^{7Y}: gemeinsam ein -(C₂-C₄)Alkandiyl-, oder (C₂-C₆)Alkendiylrest darstellen, oder
- R^{7Y}und R⁶: gemeinsam eine Bindung, ein -(C₁-C₄)Alkandiyl- oder einen (C₂-C₆)Alkendiylrest darstellen, und R^{7X} hat die oben gegebenen Bedeutung;
wobei die geschlängelten Linien an R^{5X}, R^{5Y}, R⁶, R^{7X} und R^{7Y} bedeuten, daß diese Substituenten α- oder β-ständig sein können.

Substituenten an Doppelbindungen können cis(Z)- oder trans(E)-ständig angeordnet sein.

Die Verbindungen können racemisch oder enantiomeren-rein vorliegen.

Die erfindungsgemäßen Verbindungen sind als ER-Modulatoren für die Behandlung von Estrogendefizienz bedingten Erkrankungen (altersbedingt, chirurgisch- oder medikamentös bedingt), zur Prävention und Therapie entzündlicher Erkrankungen, zur Verbesserung der Fertilität und zur Therapie von Fertilitätsstörungen der Frau sowie als Komponente neuartiger Empfängnisverhütungsmittel geeignet.

Beispielsweise können weiteren Ausführungsformen der Verbindungen der allgemeinen Formel (I) im Sinne der vorliegender Erfindung bei den abhängigen Anspruche 2 bis 32 definiert werden.

Bevorzugt gemäß vorliegender Erfindung sind die nachstehenden Verbindungen der allgemeinen Formel I:
1) 6-(4-Hydroxy-phenyl)-8,9-dihydro-7H-benzocyclohepten-2-ol
2) 6-(3-Hydroxy-phenyl)-8,9-dihydro-7H-benzocyclohepten-2-ol
3) 6-Phenyl-8,9-dihydro-7H-benzocyclohepten-2-ol
4) 6-Phenyl-5-methyl-8,9-dihydro-7H-benzocyclohepten-2-ol
5) 3-Methoxy-9-methyl-8-phenyl-6,7-dihydro-5H-benzocyclohepten
6) 6-Phenyl-5-ethyl-8,9-dihydro-7H-benzocyclohepten-2-ol
7) 9-Ethyl-3-methoxy-8-phenyl-6,7-dihydro-5H-benzocyclohepten
8) 6-Phenyl-5-propyl-8,9-dihydro-7H-benzocyclohepten-2-ol
9) 3-Methoxy-8-phenyl-9-propyl-6,7-dihydro-5H-benzocyclohepten
10) 6-Phenyl-5-butyl-8,9-dihydro-7H-benzocyclohepten-2-ol
11) 9-Butyl-3-methoxy-8-phenyl-6,7-dihydro-5H-benzocyclohepten
12) 6-(4-Hydroxy-phenyl)-5-methyl-8,9-dihydro-7H-benzocyclohepten-2-ol
13) 3-Methoxy-8-(4-methoxy-phenyl)-9-methyl-6,7-dihydro-5H-benzocyclohepten
14) 4-(2-Methoxy-5-methyl-8,9-dihydro-7H-benzocyclohepten-6-yl)-phenol
15) 6-(4-Methoxy-phenyl)-5-methyl-8,9-dihydro-7H-benzocyclohepten-2-ol
16) 6-(3-Hydroxy-phenyl)-5-methyl-8,9-dihydro-7H-benzocyclohepten-2-ol
17) 5-Ethyl-6-(4-hydroxy-phenyl)-8,9-dihydro-7H-benzocyclohepten-2-ol
18) 9-Ethyl-3-methoxy-8-(4-methoxy-phenyl)-6,7-dihydro-5H-benzocyclohepten
19) 4-(5-Ethyl-2-methoxy-8,9-dihydro-7H-benzocyclohepten-6-yl)-phenol
20) 5-Ethyl-6-(4-methoxy-phenyl)-8,9-dihydro-7H-benzocyclohepten-2-ol
21) 6-(4-Hydroxy-phenyl)-5-propyl-8,9-dihydro-7H-benzocyclohepten-2-ol
22) 3-Methoxy-8-(4-methoxy-phenyl)-9-propyl-6,7-dihydro-5H-benzocyclohepten
23) 4-(2-Methoxy-5-propyl-8,9-dihydro-7H-benzocyclohepten-6-yl)-phenol
24) 6-(4-Methoxy-phenyl)-5-propyl-8,9-dihydro-7H-benzocyclohepten-2-ol
25) 6-(4-Hydroxy-phenyl)-5-butyl-8,9-dihydro-7H-benzocyclohepten-2-ol
26) 6-(3-Hydroxy-phenyl)-5-ethyl-8,9-dihydro-7H-benzocyclohepten-2-ol
27) 6-(4-Hydroxy-phenyl)-5-trifluormethyl-8,9-dihydro-7H-benzocyclohepten-2-ol
28) 6-(4-Hydroxy-phenyl)-5-pentafluorethyl-8,9-dihydro-7H-benzocyclohepten-2-ol
29) 6-(4-Hydroxy-phenyl)-5-vinyl-8,9-dihydro-7H-benzocyclohepten-2-ol
30) 3-Methoxy-8-(4-methoxy-phenyl)-9-vinyl-6,7-dihydro-5H-benzocyclohepten
31) 6-(3-Hydroxy-phenyl)-5-vinyl-8,9-dihydro-7H-benzocyclohepten-2-ol
32) 6-(4-Hydroxy-phenyl)-6,7,8,9-tetrahydro-5H-benzocyclohepten-2-ol
33) 6-(3-Hydroxy-phenyl)-6,7,8,9-tetrahydro-5H-benzocyclohepten-2-ol
34) 6-(2-Hydroxy-phenyl)-6,7,8,9-tetrahydro-5H-benzocyclohepten-2-ol
35) 6-(4-Hydroxy-phenyl)-5-methyl-6,7,8,9-tetrahydro-5H-benzocyclohepten-2-ol
36) 6-(4-Hydroxy-phenyl)-5-ethyl-6,7,8,9-tetrahydro-5H-benzocyclohepten-2-ol
37) 6-(4-Hydroxy-phenyl)-6-methyl-6,7,8,9-tetrahydro-5H-benzocyclohepten-2-ol
38) 2-Methoxy-6-(4-methoxy-phenyl)-6-methyl-6,7,8,9-tetrahydro-5H-benzocyclohepten
39) 4-(2-Methoxy-6-methyl-6,7,8,9-tetrahydro-5H-benzocyclohepten-6-yl)-phenol
40) 6-(4-Methoxy-phenyl)-6-methyl-6,7,8,9-tetrahydro-5H-benzocyclohepten-2-ol
41) 6-(4-Hydroxy-phenyl)-6-ethyl-6,7,8,9-tetrahydro-5H-benzocyclohepten-2-ol
42) 6-Ethyl-2-methoxy-6-(4-methoxy-phenyl)-6,7,8,9-tetrahydro-5H-benzocyclohepten
43) 4-(6-Ethyl-2-methoxy-6,7,8,9-tetrahydro-5H-benzocyclohepten-6-yl)-phenol
44) 6-Ethyl-6-(4-methoxy-phenyl)-6,7,8,9-tetrahydro-5H-benzocyclohepten-2-ol
45) 6-(4-Hydroxy-phenyl)-6-vinyl-6,7,8,9-tetrahydro-5H-benzocyclohepten-2-ol
46) 2-Methoxy-6-(4-methoxy-phenyl)-6-vinyl-6,7,8,9-tetrahydro-5H-benzocyclohepten
47) 4-(2-Methoxy-6-vinyl-6,7,8,9-tetrahydro-5H-benzocyclohepten-6-yl)-phenol
48) 6-(4-Methoxy-phenyl)-6-vinyl-6,7,8,9-tetrahydro-5H-benzocyclohepten-2-ol
49) 6-(4-Hydroxy-phenyl)-4-(2-trimethylsilanyl-ethyl)-6,7,8,9-tetrahydro-5H-benzocyclohepten-2-ol
50) 6-(4-Hydroxy-phenyl)-4-(2-methyldimethoxysilanyl-ethyl)-6,7,8,9-tetrahydro-5H-benzocyclohepten-2-ol
51) 6-(4-Hydroxy-phenyl)-4-(2-triethoxysilanyl-ethyl)-6,7,8,9-tetrahydro-5H-benzocyclohepten-2-ol
52) 6-(4-Hydroxy-phenyl)-4-vinyl-6,7,8,9-tetrahydro-5H-benzocyclohepten-2-ol
53) 3-Hydroxy-8-(4-hydroxy-phenyl)-6,7,8,9-tetrahydro-5H-benzocyclohepten-1-carbaldehyd
54) 3-Hydroxy-8-(4-hydroxy-phenyl)-6,7,8,9-tetrahydro-5H-benzocyclohepten-1-carbonitril
55) 6-(4-Hydroxy-phenyl)-4-ethyl-6,7,8,9-tetrahydro-5H-benzocyclohepten-2-ol
56) 6-(4-Hydroxy-phenyl)-4-(2-fluorethyl)-6,7,8,9-tetrahydro-5H-benzocyclohepten-2-ol
57) 6-(4-Hydroxy-phenyl)-4-chlor-6,7,8,9-tetrahydro-5H-benzocyclohepten-2-ol
58) 6-(4-Hydroxy-phenyl)-5-methylen-6,7,8,9-tetrahydro-5H-benzocyclohepten-2-ol
59) 6-(4-Hydroxy-phenyl)-5,5-spirooxiran-6,7,8,9-tetrahydro-5H-benzocyclohepten-2-ol
60) 6-(4-Hydroxy-phenyl)-5,5-spirocyclopropyl-6,7,8,9-tetrahydro-5H-benzocyclohepten-2-ol
61) 5-Ethyl-6-(4-hydroxy-phenyl)-6-methyl-6,7,8,9-tetrahydro-5H-benzocyclohepten-2-ol
62) 5-Ethyl-6-(4-hydroxy-phenyl)-6-ethyl-6,7,8,9-tetrahydro-5H-benzocyclohepten-2-ol
63) 5-Hydroxy-2-(2-hydroxy-8,9-dihydro-7H-benzocyclohepten-6-yl)-benzonitril
64) 6-(2-Chloro-4-hydroxy-phenyl)-8,9-dihydro-7H-benzocyclohepten-2-ol
65) 3-Fluor-6-(4-hydroxy-phenyl)-8,9-dihydro-7H-benzocyclohepten-2-ol
66) 6-(3-Fluoro-4-hydroxy-phenyl)-8,9-dihydro-7H-benzocyclohepten-2-ol
67) 6-(2-Fluoro-4-hydroxy-phenyl)-8,9-dihydro-7H-benzocyclohepten-2-ol
68) 3-Fluoro-4-(2-methoxy-8,9-dihydro-7H-benzocyclohepten-6-yl)-phenol
69) 6-(2,3-Difluoro-4-hydroxy-phenyl)-8,9-dihydro-7H-benzocyclohepten-2-ol
70) 6-(2-Chloro-3-fluoro-4-hydroxy-phenyl)-8,9-dihydro-7H-benzocyclohepten-2-ol
71) 6-(2,5-Difluoro-4-hydroxy-phenyl)-8,9-dihydro-7H-benzocyclohepten-2-ol
72) 6-(3,5-Difluoro-4-hydroxy-phenyl)-8,9-dihydro-7H-benzocyclohepten-2-ol
73) 6-(2,6-Difluoro-4-hydroxy-phenyl)-8,9-dihydro-7H-benzocyclohepten-2-ol
74) 6-(3-Chloro-4-hydroxy-phenyl)-8,9-dihydro-7H-benzocyclohepten-2-ol
75) 6-(2-Methyl-4-hydroxy-phenyl)-8,9-dihydro-7H-benzocyclohepten-2-ol
76) 6-(2-Ethyl-4-hydroxy-phenyl)-8,9-dihydro-7H-benzocyclohepten-2-ol
77) 6-(2-Vinyl-4-hydroxy-phenyl)-8,9-dihydro-7H-benzocyclohepten-2-ol
78) 6-(2-Trifluormethyl-4-hydroxy-phenyl)-8,9-dihydro-7H-benzocyclohepten-2-ol
79) 6-(2-Chloro-4-hydroxy-phenyl)-5-methyl-8,9-dihydro-7H-benzocyclohepten-2-ol
80) 3-Fluor-6-(4-hydroxy-phenyl)-5-methyl-8,9-dihydro-7H-benzocyclohepten-2-ol
81) 6-(3-Fluoro-4-hydroxy-phenyl)-5-methyl-8,9-dihydro-7H-benzocyclohepten-2-ol
82) 6-(2-Fluoro-4-hydroxy-phenyl)-5-methyl-8,9-dihydro-7H-benzocyclohepten-2-ol
83) 6-(2,3-Difluoro-4-hydroxy-phenyl)-5-methyl-8,9-dihydro-7H-benzocyclohepten-2-ol
84) 6-(2-Chloro-3-fluoro-4-hydroxy-phenyl)-5-methyl-8,9-dihydro-7H-benzocyclohepten-2-ol
85) 6-(2,5-Difluoro-4-hydroxy-phenyl)-5-methyl-8,9-dihydro-7H-benzocyclohepten-2-ol
86) 6-(3,5-Difluoro-4-hydroxy-phenyl)-5-methyl-8,9-dihydro-7H-benzocyclohepten-2-ol
87) 6-(2,6-Difluoro-4-hydroxy-phenyl)-5-methyl-8,9-dihydro-7H-benzocyclohepten-2-ol
88) 6-(3-Chloro-4-hydroxy-phenyl)-5-methyl-8,9-dihydro-7H-benzo-cyclohepten-2-ol
89) 6-(2-Methyl-4-hydroxy-phenyl)-5-methyl-8,9-dihydro-7H-benzo-cyclohepten-2-ol
90) 6-(2-Ethyl-4-hydroxy-phenyl)-5-methyl-8,9-dihydro-7H-benzocyclohepten-2-ol
91) 6-(2-Vinyl-4-hydroxy-phenyl)-5-methyl-8,9-dihydro-7H-benzocyclohepten-2-ol
92) 6-(2-Trifluormethyl-4-hydroxy-phenyl)-5-methyl-8,9-dihydro-7H-benzocyclohepten-2-ol
93) 5-Hydroxy-2-(2-hydroxy-5-methyl-8,9-dihydro-7H-benzocyclohepten-6-yl)-benzonitril
94) 6-(2-Chloro-4-hydroxy-phenyl)- 5-ethyl-8,9-dihydro-7H-benzocyclohepten-2-ol
95) 5-Ethyl-3-fluor-6-(4-hydroxy-phenyl)-8,9-dihydro-7H-benzocyclohepten-2-ol
96) 5-Ethyl-6-(3-Fluoro-4-hydroxy-phenyl)-8,9-dihydro-7H-benzocyclohepten-2-ol
97) 6-(2-Fluoro-4-hydroxy-phenyl)- 5-ethyl-8,9-dihydro-7H-benzocyclohepten-2-ol
98) 6-(2,3-Difluoro-4-hydroxy-phenyl)-5-ethyl-8,9-dihydro-7H-benzocyclohepten-2-ol
99) 6-(2-Chloro-3-fluoro-4-hydroxy-phenyl)-5-ethyl-8,9-dihydro-7H-benzocyclohepten-2-ol
100) 6-(2,5-Difluoro-4-hydroxy-phenyl)-5-ethyl-8,9-dihydro-7H-benzocyclohepten-2-ol
101) 6-(3,5-Difluoro-4-hydroxy-phenyl)-5-ethyl-8,9-dihydro-7H-benzocyclohepten-2-ol
102) 6-(2,6-Difluoro-4-hydroxy-phenyl)-5-ethyl-8,9-dihydro-7H-benzocyclohepten-2-ol
103) 6-(3-Chloro-4-hydroxy-phenyl)-5-ethyl-8,9-dihydro-7H-benzocyclohepten-2-ol
104) 6-(2-Methyl-4-hydroxy-phenyl)-5-ethyl-8,9-dihydro-7H-benzocyclohepten-2-ol
105) 6-(2-Ethyl-4-hydroxy-phenyl)-5-ethyl-8,9-dihydro-7H-benzocyclohepten-2-ol
106) 6-(2-Vinyl-4-hydroxy-phenyl)-5-ethyl-8,9-dihydro-7H-benzocyclohepten-2-ol
107) 6-(2-Trifluormethyl-4-hydroxy-phenyl)-5-ethyl-8,9-dihydro-7H-benzocyclohepten-2-ol
108) 5-Hydroxy-2-(2-hydroxy-5-ethyl-8,9-dihydro-7H-benzocyclohepten-6-yl)-benzonitril
109) 5-Hydroxy-2-(2-hydroxy-5-propyl-8,9-dihydro-7H-benzocyclohepten-6-yl)-benzonitril
110) 6-(2-Chloro-4-hydroxy-phenyl)-5-propyl-8,9-dihydro-7H-benzocyclohepten-2-ol
111) 3-Fluoro-6-(4-hydroxy-phenyl)-5-propyl-8,9-dihydro-7H-benzocyclohepten-2-ol
112) 6-(3-Fluoro-4-hydroxy-phenyl)-5-propyl-8,9-dihydro-7H-benzocyclohepten-2-ol
113) 6-(2-Fluoro-4-hydroxy-phenyl)-5-propyl-8,9-dihydro-7H-benzocyclohepten-2-ol
114) 6-(2,3-Difluoro-4-hydroxy-phenyl)-5-propyl-8,9-dihydro-7H-benzocyclohepten-2-ol
115) 6-(2-Chloro-3-fluoro-4-hydroxy-phenyl)-5-propyl-8,9-dihydro-7H-benzocyclohepten-2-ol
116) 6-(2,5-Difluoro-4-hydroxy-phenyl)-5-propyl-8,9-dihydro-7H-benzocyclohepten-2-ol
117) 6-(3,5-Difluoro-4-hydroxy-phenyl)-5-propyl-8,9-dihydro-7H-benzocyclohepten-2-ol
118) 6-(2,6-Difluoro-4-hydroxy-phenyl)-5-propyl-8,9-dihydro-7H-benzocyclohepten-2-ol
119) 6-(3-Chloro-4-hydroxy-phenyl)-5-propyl-8,9-dihydro-7H-benzocyclohepten-2-ol
120) 6-(4-Hydroxy-2-methyl-phenyl)-5-propyl-8,9-dihydro-7H-benzocyclohepten-2-ol
121) 6-(2-Ethyl-4-hydroxy-phenyl)-5-propyl-8,9-dihydro-7H-benzocyclohepten-2-ol
122) 6-(4-Hydroxy-2-vinyl-phenyl)-5-propyl-8,9-dihydro-7H-benzocyclohepten-2-ol
123) 6-(4-Hydroxy-2-trifluoromethyl-phenyl)-5-propyl-8,9-dihydro-7H-benzocyclohepten-2-ol
124) 6-(2-Chloro-4-hydroxy-phenyl)-6-methyl-6,7,8,9-tetrahydro-5H-benzocyclohepten-2-ol
125) 3-Fluoro-6-(4-hydroxy-phenyl)-6-methyl-6,7,8,9-tetrahydro-5H-benzocyclohepten-2-ol
126) 6-(3-Fluoro-4-hydroxy-phenyl)-6-methyl-6,7,8,9-tetrahydro-5H-benzocyclohepten-2-ol
127) 6-(2-Fluoro-4-hydroxy-phenyl)-6-methyl-6,7,8,9-tetrahydro-5H-benzocyclohepten-2-ol
128) 6-(2,3-Difluoro-4-hydroxy-phenyl)-6-methyl-6,7,8,9-tetrahydro-5H-benzocyclohepten-2-ol
129) 6-(2-Chloro-3-fluoro-4-hydroxy-phenyl)-6-methyl-6,7,8,9-tetrahydro-5H-benzocyclohepten-2-ol
130) 6-(2,5-Difluoro-4-hydroxy-phenyl)-6-methyl-6,7,8,9-tetrahydro-5H-benzocyclohepten-2-ol
131) 6-(3,5-Difluoro-4-hydroxy-phenyl)-6-methyl-6,7,8,9-tetrahydro-5H-benzocyclohepten-2-ol
132) 6-(2,6-Difluoro-4-hydroxy-phenyl)-6-methyl-6,7,8,9-tetrahydro-5H-benzocyclohepten-2-ol
133) 6-(3-Chloro-4-hydroxy-phenyl)-6-methyl-6,7,8,9-tetrahydro-5H-benzocyclohepten-2-ol
134) 6-(2-Methyl-4-hydroxy-phenyl)-6-methyl-6,7,8,9-tetrahydro-5H-benzocyclohepten-2-ol
135) 6-(2-Ethyl-4-hydroxy-phenyl)-6-methyl-6,7,8,9-tetrahydro-5H-benzocyclohepten-2-ol
136) 6-(2-Vinyl-4-hydroxy-phenyl)-6-methyl-6,7,8,9-tetrahydro-5H-benzocyclohepten-2-ol
137) 6-(2-Trifluormethyl-4-hydroxy-phenyl)-6-methyl-6,7,8,9-tetrahydro-5H-benzocyclohepten-2-ol
138) 5-Hydroxy-2-(2-hydroxy-6-methyl-6,7,8,9-tetrahydro-5H-benzocyclohepten-6-yl)-benzonitril
139) 6-(2-Chloro-4-hydroxy-phenyl)-6-ethyl-6,7,8,9-tetrahydro-5H-benzocyclohepten-2-ol
140) 3-Fluoro-6-(4-hydroxy-phenyl)-6-ethyl-6,7,8,9-tetrahydro-5H-benzocyclohepten-2-ol
141) 6-(3-Fluoro-4-hydroxy-phenyl)-6-ethyl-6,7,8,9-tetrahydro-5H-benzocyclohepten-2-ol
142) 6-(2-Fluoro-4-hydroxy-phenyl)-6-ethyl-6,7,8,9-tetrahydro-5H-benzocyclohepten-2-ol
143) 6-(2,3-Difluoro-4-hydroxy-phenyl)-6-ethyl-6,7,8,9-tetrahydro-5H-benzocyclohepten-2-ol
144) 6-(2-Chloro-3-fluoro-4-hydroxy-phenyl)-6-ethyl-6,7,8,9-tetrahydro-5H-benzocyclohepten-2-ol
145) 6-(2,5-Difluoro-4-hydroxy-phenyl)-6-ethyl-6,7,8,9-tetrahydro-5H-benzocyclohepten-2-ol
146) 6-(3,5-Difluoro-4-hydroxy-phenyl)-6-ethyl-6,7,8,9-tetrahydro-5H-benzocyclohepten-2-ol
147) 6-(2,6-Difluoro-4-hydroxy-phenyl)-6-ethyl-6,7,8,9-tetrahydro-5H-benzocyclohepten-2-ol
148) 6-(3-Chloro-4-hydroxy-phenyl)-6-ethyl-6,7,8,9-tetrahydro-5H-benzocyclohepten-2-ol
149) 6-(2-Methyl-4-hydroxy-phenyl)-6-ethyl-6,7,8,9-tetrahydro-5H-benzocyclohepten-2-ol
150) 6-(2-Ethyl-4-hydroxy-phenyl)-6-ethyl-6,7,8,9-tetrahydro-5H-benzocyclohepten-2-ol
151) 6-(2-Vinyl-4-hydroxy-phenyl)-6-ethyl-6,7,8,9-tetrahydro-5H-benzocyclohepten-2-ol
152) 6-(2-Trifluormethyl-4-hydroxy-phenyl)-6-ethyl-6,7,8,9-tetrahydro-5H-benzocyclohepten-2-ol
153) 5-Hydroxy-2-(2-hydroxy-6-ethyl-6,7,8,9-tetrahydro-5H-benzocyclohepten-6-yl)-benzonitril
154) 6-(2-Chloro-4-hydroxy-phenyl)-6,7,8,9-tetrahydro-5H-benzocyclohepten-2-ol
155) 3-Fluoro-6-(4-hydroxy-phenyl)-6,7,8,9-tetrahydro-5H-benzocyclohepten-2-ol
156) 6-(3-Fluoro-4-hydroxy-phenyl)-6,7,8,9-tetrahydro-5H-benzocyclohepten-2-ol
157) 6-(2-Fluoro-4-hydroxy-phenyl)-6,7,8,9-tetrahydro-5H-benzocyclohepten-2-ol
158) 6-(2,3-Difluoro-4-hydroxy-phenyl)-6,7,8,9-tetrahydro-5H-benzocyclohepten-2-ol
159) 6-(2-Chloro-3-fluoro-4-hydroxy-phenyl)-6,7,8,9-tetrahydro-5H-benzocyclohepten-2-ol
160) 6-(2,5-Difluoro-4-hydroxy-phenyl)-6,7,8,9-tetrahydro-5H-benzocyclohepten-2-ol
161) 6-(3,5-Difluoro-4-hydroxy-phenyl)-6,7,8,9-tetrahydro-5H-benzocyclohepten-2-ol
162) 6-(2,6-Difluoro-4-hydroxy-phenyl)-6,7,8,9-tetrahydro-5H-benzocyclohepten-2-ol
163) 6-(3-Chloro-4-hydroxy-phenyl)-6,7,8,9-tetrahydro-5H-benzocyclohepten-2-ol
164) 6-(2-Methyl-4-hydroxy-phenyl)-6,7,8,9-tetrahydro-5H-benzocyclohepten-2-ol
165) 6-(2-Ethyl-4-hydroxy-phenyl)-6,7,8,9-tetrahydro-5H-benzocyclohepten-2-ol
166) 6-(2-Vinyl-4-hydroxy-phenyl)-6,7,8,9-tetrahydro-5H-benzocyclohepten-2-ol
167) 6-(2-Trifluormethyl-4-hydroxy-phenyl)-6,7,8,9-tetrahydro-5H-benzocyclohepten-2-ol
168) 5-Hydroxy-2-(2-hydroxy-6,7,8,9-tetrahydro-5H-benzocyclohepten-6-yl)-benzonitril
169) 5-Allyl-6-(4-hydroxy-phenyl)-8,9-dihydro-7H-benzocyclohepten-2-ol
170) 9-Allyl-3-methoxy-8-(4-methoxy-phenyl)-6,7-dihydro-5H-benzocyclohepten
171) 6-Allyl-2-methoxy-6-(4-methoxy-phenyl)-6,7,8,9-tetrahydro-benzocyclohepten-5-on
172) 6-Allyl-2-methoxy-6-(4-methoxy-phenyl)-6,7,8,9-tetrahydro-5H-benzocyclohepten
173) 6-Allyl-6-(4-hydroxy-phenyl)-6,7,8,9-tetrahydro-5H-benzocyclohepten-2-ol
174) 2-Methoxy-6-(4-methoxy-phenyl)-6,7,8,9-tetrahydro-benzocyclohepten-5-on
175) 2-Methoxy-6-(4-methoxy-phenyl)-6-propyl-6,7,8,9-tetrahydro-benzocyclohepten-5-on
176) 2-Methoxy-6-(4-methoxy-phenyl)-6-propyl-6,7,8,9-tetrahydro-5H-benzocyclohepten
177) 6-(4-Hydroxy-phenyl)-6-propyl-6,7,8,9-tetrahydro-5H-benzocyclohepten-2-ol
178) 5-Allyl-6-(4-hydroxy-phenyl)-6-methyl-6,7,8,9-tetrahydro-5H-benzocyclohepten-2-ol
179) 5-Allyl-6-(4-hydroxy-phenyl)-6-ethyl-6,7,8,9-tetrahydro-5H-benzocyclohepten-2-ol
180) 6-(4-Hydroxy-phenyl)-4-trifluoromethyl-6,7,8,9-tetrahydro-5H-benzocyclohepten-2-ol
181) 4,6-Diethyl-6-(4-Hydroxy-phenyl)-6,7,8,9-tetrahydro-5H-benzocyclohepten-2-ol
182) 4-Ethyl-6-(4-Hydroxy-phenyl)-6-methyl-6,7,8,9-tetrahydro-5H-benzocyclohepten-2-ol
183) 6-(3-Hydroxy-phenyl)-6-methyl-6,7,8,9-tetrahydro-5H-benzocyclohepten-2-ol
184) 6-(3-Hydroxy-phenyl)-5-butyl-6,7,8,9-tetrahydro-5H-benzocyclohepten-2-ol
185) 6-(4-Hydroxy-phenyl)-5-propyl-6,7,8,9-tetrahydro-5H-benzocyclohepten-2-ol
186) 6-(3-Hydroxy-phenyl)-5-propyl-6,7,8,9-tetrahydro-5H-benzocyclohepten-2-ol
187) 6-(4-Hydroxy-phenyl)-5-propenyl-8,9-dihydro-7H-benzocyclohepten-2-ol
188) 6-(4-Hydroxy-phenyl)-5,5-dimethyl-8,9-dihydro-5H-benzocyclohepten-2-ol
189) 6-(4-Hydroxy-phenyl)-5,5-dimethyl-6,7,8,9-tetrahydro-5H-benzocyclohepten-2-ol
190) 5-Ethyl-6-(4-hydroxy-phenyl)-5-methyl-8,9-dihydro-5H-benzocyclohepten-2-ol
191) 5,5-Diethyl-6-(4-hydroxy-phenyl)-8,9-dihydro-5H-benzocyclohepten-2-ol
192) 6-(4-Hydroxyphenyl)-5-phenyl-8,9-dihydro-7H-benzocyclohepten-2-ol
193) 6-(4-Hydroxyphenyl)-5-(3-methylthiophen-2-yl)-8,9-dihydro-7H-benzocyclohepten-2-ol

### Herstellung der erfindungsgemässen Verbindungen

Benzocycloheptenderivate gemäß vorliegender Erfindung können mit dem folgenden Syntheseweg hergestellt werden:

2-Methoxy-5,7,8,9-tetrahydro-benzocyclohepten-6-on wird nach bekannten Methoden aus 6-Methoxytetralon durch Olefinierung z.B. mit Methyltriphenylphosphonium-bromid/Base, wie z.B. KOt.-Bu und Umlagerung mit TI(NO₃)₃ (TTN), [Hydroxy(tosyloxy)iodo]benzen HTIB oder AgNO₃/I₂ gewonnen. Die Mono- oder Dialkylierung des 6-Methoxytetralon vor der Wittig-Reaktion und der TTN-Umlagerung führen zu den entsprechenden 7-substituierten 2-Methoxybenzocycloheptan-6-onen.
Die Verwendung von höheren Alkyltriphenylphosphoniumbromiden in der Wittig-Reaktion führt nach TTN-Umlagerung zu entsprechenden 5-substituierten 2-Methoxybenzocycloheptan-6-onen. [E.C. Taylor et al., Tetrahed. Letters, 1977, 1827-1830; A. I. Khalaf et al., J. Chem. Soc. Perkin Trans. 1, 12, 1992, 1475-1482; C. M. M. da Conceicao et al., J. Chem. Res. Miniprint, 9, 1995, 2161-2194.]

Die unsubstituierten und 5- bzw. 7-substituierten 2-Methoxy-5,7,8,9-tetrahydro-benzocyclohepten-6-one können anschließend durch weitere Alkylierung mittels Alkylhalogeniden und einer Base wie z.B. KOt.-Bu die entsprechenden höher substituierten 2-Methoxy-5,7,8,9-tetrahydro-benzocyclohepten-6-one hergestellt werden.

Die Dialkylierung unsubstituierter 2-Methoxy-5,7,8,9-tetrahydro-benzocyclohepten-6-one mittels Alkyldihalogeniden führt zu Spiroverbindungen.

Die unsubstituierten oder alkylsubstituierten 2-Methoxy-5,7,8,9-tetrahydro-benzocyclohepten-6-one werden in eine Enolverbindung wie z.B. Enoltriflat oder Enolnonaflat überführt. Die anschließende Palladium-katalysierte Kupplung (z.B. Suzuki-Kupplung) mit substituierten Boronsäuren führt zur Bildung der erfindungsgemäßen Verbindungen, den Benzocycloheptenen gemäß Formel I. Mittels Hydroborierung und anschließender Oxidation werden die Verbindungen in die entsprechenden Benzocycloheptan-5-one überführt.

Die hergestellten Benzocyclohepten-5-one können durch bekannte Reaktionen wie z.B:
- Reduktionen mit Hydriden (z.B. Borhydride, Boranate, Alanate) oder metallkatalysiert mit Wasserstoff (z.B. Pd-, Pt-Katalyse)
- Dehydratisierung (z.B. säurekatalysiert)
- 6-Alkylierung, 5-Alkylierung (z.B. mit Alkylhalogeniden)
- Deoxigenierung (z.B. mit Silanen)
- Enolisierung in Position 5 (z.B. als Triflat oder Nonaflat) und Palladiumkupplung (z.B. Stille- oder Suzuki-Kupplung)
- Ruthenium katalysierte 4-Alkylierung
- Olefinierung in Position 5 (z.B. Wittig-Olefinierung)
- Etherspaltung (z.B. Borhalogenide für Methylether)
   in die erfindungsgemäßen Verbindungen der Formel I umgewandelt werden, wobei die eingeführten Substituenten bzw. Gruppen durch die dem Fachmann bekannten chemischen Reaktionen weiter umgewandelt werden können.

### Definition der Substituenten in den Verbindungen der Formel I:

Unter "(C₁-C₄)Alkylrest" wird im Sinne der vorliegenden Erfindung ein verzweigter oder geradkettiger Alkylrest mit 1 bis 4 Kohlenstoffatomen verstanden, der mit F, Cl, Br, Hydroxygruppen oder (C₁-C₄)Alkyl-O-rest (OMe) oder CN substituiert sein kann. Als Ausführungsbeispiele seien ein Methyl-, Ethyl-, n-Propyl-, i-Propyl-, n-Butyl-, i-Butyl-, tert.-Butyl-, 1-Bromoethyl-, 2-Bromoethyl-, 1-Chloroethyl-, 2-Chloroethyl-, 1-Fluoroethyl-, 2-Fluoroethylrest genannt. Im Sinne der vorliegenden Erfindung sind Methyl, Ethyl, Propyl und Isopropyl für den (C₁-C₄)-Alkylrest bevorzugt.

Unter "(C₃-C₆)Cyclo-alkylrest" wird im Sinne der vorliegenden Erfindung ein carbocyclischer Rest mit 3 bis 6 Kohlenstoffatomen verstanden, der mit F, Cl, Br, Hydroxygruppen oder (C₁-C₄)Alkyl-O-rest (OMe) oder CN substituiert sein kann. Als Ausführungsbeispiele seien der Cyclopropyl-, Cyclopentyl- und der Cyclohexylrest genannt. Im Sinne der vorliegenden Erfindung ist der Cyclopentylrest für den (C₃-C₆)Cyclo-alkylrest bevorzugt.

Unter "(C₁-C₁₄)Acylrest" wird im Sinne der vorliegenden Erfindung ein aliphatischer, gerad- oder verzweigtkettiger, gesättigter oder ungesättigter (C₁-C₁₄)-Carbonylrest, ein aromatischer Carbonylrest oder der aus einer α-oder β-Aminosäure abgeleitete Carbonylrest verstanden, der mit F, Cl oder Br substituiert sein kann. Als derartige Reste kommen beispielsweise die aus folgenden Carbonsäuren abgeleiteten Carbonylreste in Betracht: Ameisensäure, Essigsäure, Propionsäure, Buttersäure, Isobuttersäure, Valeriansäure, Isovaleriansäure, Pivalinsäure, Laurinsäure, Myristinsäure, Acrylsäure, Propiolsäure, Methacrylsäure, Crotonsäure, Isocrotonsäure, Ölsäure, Elaidinsäure. Dicarbonsäuren: Oxalsäure, Malonsäure, Bernsteinsäure, Glutarsäure, Adipinsäure, Pimelinsäure, Korksäure, Azelainsäure, Sebacinsäure, Maleinsäure, Fumarsäure, Muconsäure, Citraconsäure, Mesaconsäure, Benzoesäure, Phthalsäure, Isophthalsäure, Terephthalsäure, Naphthoesäure, o-, m- und p-Toluylsäure, Hydratropasäure, Atropasäure, Zimtsäure, Nicotinsäure, Isonicotinsäure, Alanin, β-Alanin, Arginin, Cystein, Cystin, Glycin, Histidin, Leucin, Isoleucin, Phenylalanin, Prolin.

Unter "(C₁-C₄)-Alkenylrest" wird im Sinne der vorliegenden Erfindung ein verzweigter oder geradkettiger Alkylrest mit 1 bis 4 Kohlenstoffatomen verstanden, der mit F, Cl, Br, Hydroxygruppen oder (C₁-C₄)Alkyl-O-rest (OMe) oder CN substituiert sein kann. Als Ausführungsbeispiele der vorliegenden Erfindung seien ein Vinyl-, Prop-1-enyl-, Allyl-, 2-Methylallyl-, i-Propenyl-, n-But-1-enyl-, n-But-2-enyl-, 2-Methylpropenyl-, 2-Bromovinyl-, 3-Bromopropenyl-, 2-Bromopropenyl-, 1-Bromoallylrest-, 3-Bromoallyl-, 3-Chloroallyl-, 3-Chloropropenyl-, 3-Fluoroallyl- oder 3-Fluoropropenylrest genannt.

Unter "Perfluor (C₁-C₄)-Alkylrest" wird im Sinne der vorliegenden Erfindung ein verzweigter oder geradkettiger perfluorierter Alkylrest mit 1 bis 4 Kohlenstoffatomen verstanden. Als Ausführungsbeispiele der vorliegenden Erfindung seien ein Trifluormethyl-, Pentafluorethyl-, Heptafluor-n-propyl- oder Heptafluor-iso-propylrest genannt.

Unter "(C₂-C₄)Alkandiylrest" oder "(C₁-C₄)Alkandiylrest" wird im Sinne der vorliegenden Erfindung ein verzweigter oder geradkettiger Rest mit 1 bis 4 oder 2 bis 4 Kohlenstoffatomen verstanden. Als Ausführungsbeispiele sei - CH₂-CH₂-CH₂-CH₂- genannt.

Unter "(C₂-C₆)Alkendiylrest" wird im Sinne der vorliegenden Erfindung ein verzweigter oder geradkettiger Rest mit 2 bis 6 Kohlenstoffatomen verstanden. Als Ausführungsbeispiele seien -CH₂-CH=CH-CH₂-, CH₂-CH=CH-CH₂-CH₂-, -CH₂-CH₂-CH=CH-CH₂-CH₂- genannt.

Unter "Perfluor (C₁-C₄)-Alkenylrest" wird im Sinne der vorliegenden Erfindung ein verzweigter oder geradkettiger perfluorierter Alkenylrest mit 1 bis 4 Kohlenstoffatomen verstanden, der durch Chlor und Brom substituiert sein kann. Als Ausführungsbeispiele der vorliegenden Erfindung seien ein Trifluorovinyl-, Pentafluoropropenyl-, Pentafluoroallyl-, 2-Chloro-1,2-difluoro-vinyl-, Heptafluorobut-2-enyl- oder Heptafluorobut-3-enylrest genannt.

Unter Arylrest wird ein Phenylrest verstanden, der durch F, Cl, OMe, Me, Et, OEt, CF₃ substituiert sein kann. Als Beispiele seien Phenyl, 2-Fluorphenyl, 3-Fluorphenyl, 2-Chlorphenyl, 3-Chlorphenyl, Toluenyl genannt.

Unter Heteroarylrest wird ein Thiophen-, Pyridin-, Pyrrolidin und Furanrest verstanden, der durch F, Cl, OMe, Me, Et, OEt, CF₃ substituiert sein kann. Als Ausführungsbeispiele der vorliegenden Erfindung seien Thiophen-2-yl, Thiophen-3-yl, Pyridin-2-yl, Pyridin-3-yl, Pyridin-4-yl, N-Methylpyrrolidin-2-yl, 3-Methylthiophen-2-yl und 3-Chlorthiophen-2-yl genannt.

Sowohl der Aryl- als auch der Heteroarylrest können substituiert sein.

Als Substituenten für einen Aryl- oder Heteroarylrest seien zum Beispiel ein Methyl-, Ethyl-, Trifluormethyl- Pentafluorethyl-, Trifluormethylthio-, Methoxy-, Ethoxy-, Nitro-, Cyano-, Halogen- (Fluor, Chlor, Brom, lod), Hydroxy-, Amino-, Mono((C₁-C₄)alkyl)-oder Di((C₁-C₄)alkyl)amino, wobei beide Alkylgruppen identisch oder verschieden sind, Di(aralkyl)amino, wobei beide Aralkylgruppen identisch oder verschieden sind, erwähnt.

Unter dem Begriff "Halogenatom" wird im Rahmen der vorliegenden Erfindung ein Fluor-, Chlor-, Brom- oder lodatom verstanden.

Unter (C₁-C₄)Alkinylrest wird im Sinne der vorliegenden Erfindung ein Alkinylrest mit 1 bis 4 Kohlenstoffatomen verstanden, der durch Fluor, Chlor und Brom substituiert sein kann. Als Ausführungsbeispiele der vorliegenden Erfindung seien ein Ethinyl-, Prop-1-inyl-, Prop-2-inyl-, But-2-inyl oder Trifluoroprop-1-ynylrest genannt.

Unter (C₁-C₄)Alkyl-O- rest wird im Sinne der vorliegenden Erfindung ein über Sauerstoff gebundener (C₁-C₄)Alkylrest mit 1 bis 4 Kohlenstoffatomen verstanden, der durch Fluor, Chlor und Brom substituiert sein kann. Als Ausführungsbeispiele der vorliegenden Erfindung seien ein Methoxy-, Ethoxy- oder 2-Fluoroethoxyrest genannt.

Unter "(C₃-C₆)Cyclo-alkyl-O-rest" wird im Sinne der vorliegenden Erfindung ein über Sauerstoff gebundener (C₃-C₆)Cyclo-alkyl-rest verstanden, der mit F, Cl, Br, Hydroxygruppen oder (C₁-C₄)Alkyl-O-rest (OMe) oder CN substituiert sein kann. Als Ausführungsbeispiele seien der Cyclopropyloxy-, Cyclopentyloxy- und ein Cyclohexyloxyrest genannt. Im Sinne der vorliegenden Erfindung ist der Cyclopentyloxyrest für den (C₃-C₆)Cyclo-alkyl-O-rest bevorzugt.

Unter (C₁-C₁₄)Acyl-O-rest wird im Sinne der vorliegenden Erfindung ein Rest benannt, der aus der Veresterung einer freien Hydroxylgruppe mit einer aliphatischen, gerad- oder verzweigtkettigen, gesättigten oder ungesättigten (C₁-C₁₄)Mono- oder Polycarbonsäure oder einer aromatischen Carbonsäure oder mit einer α- oder β-Aminosäure hervorgegangen ist. Als Reste kommen beispielsweise die aus folgenden Carbonsäuren abgeleiteten Carbonylreste in Betracht: Ameisensäure, Essigsäure, Propionsäure, Buttersäure, Isobuttersäure, Valeriansäure, Isovaleriansäure, Pivalinsäure, Laurinsäure, Myristinsäure, Acrylsäure, Propiolsäure, Methacrylsäure, Crotonsäure, Isocrotonsäure, Ölsäure, Elaidinsäure. Dicarbonsäuren: Oxalsäure, Malonsäure, Bernsteinsäure, Glutarsäure, Adipinsäure, Pimelinsäure, Korksäure, Azelainsäure, Sebacinsäure, Maleinsäure, Fumarsäure, Muconsäure, Citraconsäure, Mesaconsäure, Benzoesäure, Phthalsäure, Isophthalsäure, Terephthalsäure, Naphthoesäure, o-, m- und p-Toluylsäure, Hydratropasäure, Atropasäure, Zimtsäure, Nicotinsäure, Isonicotinsäure, Alanin, β-Alanin, Arginin, Cystein, Cystin, Glycin, Histidin, Leucin, Isoleucin, Phenylalanin, Prolin.

Unter "Silyl-" wird im Sinne der vorliegenden Erfindung ein Si(R)₃ benannt wobei R ein H-Atom oder ein (C₁-C₄)-Alkyl- darstellt

Freie Hydroxylgruppen in den Verbindungen der allgemeinen Formel I können mit einer aliphatischen, gerad- oder verzweigtkettigen, gesättigten oder ungesättigten (C₁-C₁₄)Mono- oder Polycarbonsäure oder einer aromatischen Carbonsäure oder mit einer α- oder β-Aminosäure verestert sein.
Als derartige Carbonsäuren zur Veresterung kommen beispielsweise in Betracht: Monocarbonsäuren: Ameisensäure, Essigsäure, Propionsäure, Buttersäure, Isobuttersäure, Valeriansäure, Isovaleriansäure, Pivalinsäure, Laurinsäure, Myristinsäure, Acrylsäure, Propiolsäure, Methacrylsäure, Crotonsäure, Isocrotonsäure, Ölsäure, Elaidinsäure.
Dicarbonsäuren: Oxalsäure, Malonsäure, Bernsteinsäure, Glutarsäure, Adipinsäure, Pimelinsäure, Korksäure, Azelainsäure, Sebacinsäure, Maleinsäure, Fumarsäure, Muconsäure, Citraconsäure, Mesaconsäure.
Aromatische Carbonsäuren: Benzoesäure, Phthalsäure, Isophthalsäure, Terephthalsäure, Naphthoesäure, o-, m- und p-Toluylsäure, Hydratropasäure, Atropasäure, Zimtsäure, Nicotinsäure, Isonicotinsäure.
Als Aminosäuren kommen die dem Fachmann hinlänglich bekannten Vertreter dieser Substanzklasse in Frage, beispielsweise Alanin, β-Alanin, Arginin, Cystein, Cystin, Glycin, Histidin, Leucin, Isoleucin, Phenylalanin, Prolin etc..

Die erfindungsgemäßen Ester der Verbindungen nach der vorliegenden Erfindung weisen als Prodrugs Vorteile gegenüber den unveresterten Wirkstoffen hinsichtlich ihres Applikationsmodus, ihrer Wirkungsart, Wirkungsstärke und Wirkungsdauer auf.

Pharmakokinetische und pharmakodynamische Vorteile weisen auch die erfindungsgemäßen Sulfamatderivate auf. Diesbezügliche Effekte wurden bereits bei Sulfamaten beschrieben, die sich von Estrogenen mit natürlicher absoluter Konfiguration ableiten (J. Steroid Biochem. Molec. Biol, 55, 395 - 403 (1995); Exp. Opinion Invest. Drugs 7, 575 - 589 (1998)).

Die Substituenten R^{5x}, R^{5y}, R⁶, R^{7x}, R^{7y} können jeweils in der α- oder β-Position stehen.

Die im vorliegenden Patent beschriebenen neuartigen Estrogene können als Einzelkomponente in pharmazeutischen Zubereitungen oder in Kombination insbesondere mit Gestagenen, Androgenen oder Antiestrogenen eingesetzt werden.

Die Substanzen und die sie enthaltenden Pharmaka eignen sich z. B. als Komponente von oralen Kontrazeptiva, etwa in Kombination mit einem Gestagen.

In der vorliegenden Patentanmeldung werden Benzocycloheptenderivate zur Behandlung von estrogendefizienz-bedingten Krankheiten und Zuständen beschrieben.

Die Erfindung betrifft auch pharmazeutische Präparate, die mindestens eine Verbindung der allgemeinen Formel I (oder physiologisch verträgliche Additionssalze mit organischen und anorganischen Säuren davon) enthalten und die Verwendung dieser Verbindungen zur Herstellung von Arzneimitteln, insbesondere für die nachstehenden Indikationen.

Die Verbindungen können, sowohl nach oraler als auch parenteraler Gabe, für die folgenden Indikationen eingesetzt werden.

Die in der vorliegenden Patentanmeldung beschriebenen neuartigen nicht-steroidalen Estrogene können als Einzelkomponente in pharmazeutischen Zubereitungen oder in Kombination insbesondere mit Antiestrogenen oder Gestagenen eingesetzt werden. Besonders bevorzugt ist die Kombination der selektiven Estrogene mit ERα-selektiven Antiestrogenen, oder mit Antiestrogenen, die peripher selektiv wirksam sind, d.h. die die Bluthirnschranke nicht passieren.

Die Substanzen und die sie enthaltenden Pharmaka sind besonders geeignet für die Behandlung peri- und postmenopausaler Beschwerden insbesondere Hitzewallungen, Schlafstörungen, Reizbarkeit, Stimmungsschwankungen, Inkontinenz, Vaginalatrophie, hormondefizienz-bedingte Gemütserkrankungen. Ebenso sind die Substanzen für die Hormonsubstitution und die Therapie von hormondefizienz-bedingten Beschwerden bei chirurgisch, medikamentös oder anders bedingter ovarieller Dysfunktion geeignet.

Die Verbindungen sind auch zur Linderung der Symptome der Andropause und Menopause, d.h. zur männlichen und weiblichen Hormonersatz-Therapie (HRT), und zwar sowohl zur Prävention als auch zur Behandlung, weiterhin zur Behandlung der mit einer Dysmenorrhoe einhergehenden Beschwerden sowie zur Behandlung der Akne geeignet.

Die Substanzen üben außerdem estrogenartige Wirkung auf das Gefäßsystem und Gehirnfunktionen aus.

Die Substanzen sind ferner zur Vorbeugung gegen Herzkreislauferkrankungen, insbesondere Gefäßerkrankungen wie Atherosklerose, zur Hemmung der Proliferation der arteriellen Glattmuskelzellen, zur Behandlung des primären pulmonaren Bluthochdrucks und zur Vorbeugung gegen hormondefizienz-bedingte neurodegenerative Erkrankungen, wie Alzheimersche Krankheit, sowie hormondefizienz-bedingte Beeinträchtigung von Gedächtnis- und Lernfähigkeit, einsetzbar.

Weiterhin sind die Substanzen zur Behandlung von entzündlichen und Erkrankungen des Immunsystems, insbesondere Autoimmunerkrankungen, wie z.B. Rheumatoide Arthritis und MS einsetzbar. Insbesondere sind die erfindungsgemäßen Substanzen zur Vorbeugung und Behandlung von Endometriose einsetzbar.

Die in der vorliegenden Patentanmeldung beschriebenen nicht-steroidale Estrogene sind außerdem zur Vorbeugung und Behandlung von Alopezie geeignet.
Die beschriebenen Substanzen sind zur Therapie von Hitzewallungen, zur Verbesserung der Ovarfunktion und Stimulierung der Follikulogenese und auch als Komponente neuartiger Kontrazeptiva geeignet.

Außerdem können die Verbindungen zur Behandlung männlicher Fertilitätsstörungen und prostatischer Erkrankungen Verwendung finden.

Die Substanzen sind außerdem zur Vorbeugung und Therapie von Estrogen abhängigen Magen-Darm-Karzinomen geeignet.

Schließlich können die Verbindungen der allgemeinen Formel I in Verbindung mit Progesteronrezeptor-Antagonisten verwendet werden, und zwar insbesondere zur Verwendung in der Hormonersatz-Therapie und zur Behandlung gynäkologischer Störungen.

Ein therapeutisches Produkt, enthaltend ein *Estrogen* und ein reines Antiestrogen für gleichzeitige, sequentielle oder getrennte Anwendung für die selektive Estrogentherapie peri- oder postmenopausaler Zustände ist bereits in der EP-A 0 346 014 beschrieben.

Die zu verabreichende Menge einer Verbindung der allgemeinen Formel I schwankt innerhalb eines weiten Bereichs und kann jede wirksame Menge abdecken. In Abhängigkeit des zu behandelnden Zustands und der Art der Verabreichung kann die Menge der verabreichten Verbindung 0,01 µg/kg - 10 mg/kg Körpergewicht, vorzugsweise 0,04 pg/kg - 1 mg/kg Körpergewicht, je Tag betragen.
Beim Menschen entspricht dies einer Dosis von 0,8 µg bis 800 mg, vorzugsweise 3,2 µg bis 80 mg, täglich.

Eine Dosiseinheit enthält erfindungsgemäß 1,6 µg bis 200 mg einer oder mehrerer Verbindungen der allgemeinen Formel I.

Die erfindungsgemäßen Verbindungen und die Säureadditionssalze sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen oder deren Säureadditionssalze, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche in Frage, die in folgenden Literaturstellen als Hilfsstoffe für Pharmazie, Kosmetik und angrenzende Gebiete empfohlen beziehungsweise angegeben sind: Ullmans Encyklopädie der technischen Chemie, Band 4 (1953), Seite 1 bis 39; Journal of Pharmaceutical Sciences, Band 52 (1963), Seite 918 ff., H. v. Czetsch-Lindenwald, Hilfsstoffe für Pharmazie und angrenzende Gebiete; Pharm. Ind., Heft 2, 1961, Seite 72 u. ff.: Dr. H. P. Fiedler, Lexikon der Hilfsstoffe für Pharmazie, Kosmetik und angrenzende Gebiete, Cantor KG. Aulendorf in Württemberg 1971.

Die Verbindungen können oral oder parenteral, beispielsweise intraperitoneal, intramuskulär, subkutan oder perkutan verabreicht werden. Die Verbindungen können auch in das Gewebe implantiert werden.
Zur oralen Verabreichung kommen Kapseln, Pillen, Tabletten, Dragees usw. in Frage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten.
Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnußöl, Baumwollsamenöl, Sojabohnenöl, Rizinusöl und Sesamöl.

Die Verbindungen lassen sich auch in Form einer Depotinjektion oder eines Implantatpräparats anwenden, die so formuliert sein können, daß eine verzögerte Wirkstoff-Freigabe ermöglicht wird.
Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

Für die Herstellung von mit aktiven Verbindungen der allgemeinen Formel I beladenen Intravaginal- (z.B. Vaginalringe) oder Intrauterinsystemen (z.B. Pessare, Spiralen, IUSs, Mirena^{®}) für die lokale Verabreichung eignen sich verschiedene Polymere wie zum Beispiel Silikonpolymere, Ethylenvinylacetat, Polyethylen oder Polypropylen.

Um eine bessere Bioverfügbarkeit des Wirkstoffes zu erreichen, können die Verbindungen auch als Cyclodextrinclathrate formuliert werden. Hierzu werden die Verbindungen mit α-, β- oder γ-Cyclodextrin oder Derivaten von diesen umgesetzt.

Erfindungsgemäß können die Verbindungen der allgemeinen Formel I auch mit Liposomen verkapselt werden.

### Methodik

### Estrogenrezeptorbindungsstudien

Die Bindungsaffinität der neuen Estrogene wurde in Kompetitionsexperimenten unter Verwendung von 3H-Estradiol als Ligand an Estrogenrezeptorpräparationen von Rattenprostata und Rattenuterus getestet. Die Präparation des Prostatacytosols und der Estrogenrezeptortest mit dem Prostatacytosol wurde, wie von Testas et al. (1981) beschrieben, durchgeführt (Testas J. et al., 1981, Endocrinology 109: 1287-1289). Die Präparation von Rattenuteruscytosol sowie der Rezeptortest mit dem ERhaltigen Cytosol wurden prinzipiell durchgeführt wie von Stack und Gorski, 1985, beschrieben (Stack, Gorski 1985, Endocrinology 117, 2024-2032) mit einigen Modifikationen wie bei Fuhrmann et al. (1995) beschrieben (Fuhrmann U. et al. 1995, Contraception 51: 45-52).

Dabei wird davon ausgegangen, daß ERβ gegenüber ERα in der Rattenprostata, in Rattenuterus ERα gegenüber ERβ überwiegt. Tabelle 1 zeigt, daß das Verhältnis der Bindung an Prostata- und Uterusrezeptor qualitativ mit dem Quotient der relativen Bindungsaffinität (RBA) an humanen ERβ und ERα von Ratte (nach Kuiper et al. (1996), Endocrinology 138: 863-870) übereinstimmt (Tabelle 1).
Weiterhin wurde die Prädiktivität des 'Prostata-ER versus Uterus-ER-Testssystems' hinsichtlich gewebeselektiver Wirkung durch in vivo Untersuchungen bestätigt. Substanzen mit Präferenz für Prostata-ER sind in vivo hinsichtlich Uteruswirkung zugunsten der Wirkung am Ovar dissoziiert.

Tabelle 2 zeigt die Ergebnisse für die erfindungsgemäß zu verwendende Verbindungen.

### Zellulärer in vitro-Test zur Bestimmung der Oestrogen Rezeptor -α und -β Aktivität

Abkürzungen:
- DMEM: Dulbecco's Modified Eagle Medium
- DNA: Desoxy Nucleic Acid
- FCS: Fetal Calf Serum
- HEPES: 4-(2-hydroxyethyl)-1-piperazineethanesulfonic acid
- PCR: Polymerase Chain Reaction

Die Identifizierung von Modulatoren der humanen Oestrogen Rezeptoren -α und -β (ERβα und ERβ) sowie die Quantifizierung der Wirksamkeit der hier beschriebenen Substanzen erfolgt mit Hilfe von rekombinanten Zelllinien. Diese Zellen leitet sich ursprünglich von einer Ovarepithelzelle des Hamsters (Chinese Hamster Ovary, CHO K1, ATCC: American Type Culture Collection, VA 20108, USA) ab.

In dieser CHO K1 Zelllinie wird ein etabliertes Chimärensystem verwendet, in dem die Liganden-Bindungsdomänen humaner Steroidhormon-Rezeptoren an die DNA-Bindungsdomäne des Hefe-Transkriptionsfaktors GAL4 fusioniert werden. Die so entstehenden GAL4-Steroidhormon-Rezeptor-Chimären werden in den CHO-Zellen mit einem Reporterkonstrukt co-transfiziert und stabil exprimiert.

### Klonierungen:

Zur Generierung der GAL4-Steroidhormonrezeptor-Chimären wird die GAL4-DNA-Bindungsdomäne (Aminosäuren 1-147) aus dem Vektor pFC2-dbd (Firma Stratagene) mit den PCR-amplifizierten Liganden-Bindungsdomänen des Oestrogen-Rezeptors α (ERα, Genbank Akzessionsnummer NM00125, Aminosäuren 282-595) und des Oestrogen-Rezeptors β (ERβ, Genbank Akzessionsnummer AB006590, Aminosäuren 223-530) in den Vektor pIRES2 (Firma Clontech) kloniert. Das Reporterkonstrukt, welches fünf Kopien der GAL4-Bindestelle, vorgeschaltet vor einem Thymidinkinasepromotor, enthält, führt zur Expression der Firefly-Luciferase (Photinus pyralis) nach Aktivierung und Bindung der GAL4-Oestrogenrezeptor-Chimären durch spezifische Agonisten.

Testablauf: Die Stammkulturen der ERα-und ERβ-Zellen werden routinemässig in DMEM/F12 Medium, 10% FCS, 1 % Hepes, 1 % Penizillin/Streptomycin, 1 mg/ml G418, sowie 5 µg/ml Puromycin kultiviert. Am Tag vor dem Test werden die ERα und ERβ-Zellen in Opti-MEM Medium (Optimem, Firma Invitrogen, 2,5% aktivkohlegereinigtes FCS der Firma Hyclone, 1 % Hepes) in 96- (oder 384) Loch-Mikrotiterplatten ausplattiert und in einem Zellinkubator (96% Luftfeuchtigkeit, 5% v/v CO₂, 37°C) gehalten. Am Testtag werden die zu prüfenden Substanzen in oben genanntem Medium aufgenommen und zu den Zellen dazugegeben. Sollen mögliche antagonistische Eigenschaften von Testsubstanzen untersucht werden, wird 10 bis 30 Minuten nach Zugabe der Testsubstanzen der Oestrogenrezeptoragonist 17-β Estradiol (Firma Sigma) hinzugegeben, bei der Untersuchung agonistischer Eigenschaften erfolgt keine zusätzliche Zugabe von 17-β Estradiol. Nach einer weiteren Inkubationszeit von 5 bis 6 Stunden werden die Zellen mit einem Luziferin/Triton-Puffer lysiert und die Luciferaseaktivität mit Hilfe einer Videokamera gemessen. Die gemessenen relativen Lichteinheiten ergeben in Abhängigkeit von der Substanzkonzentration eine sigmoide Stimulationskurve. Die Berechnung der EC₅₀- bzw. IC₅₀-Werte erfolgt mit Hilfe des Computerprogramms GraphPad PRISM (Version 3.02).

Tabelle 3 zeigt die Ergebnisse für die erfindungsgemäß zu verwendende Verbindungen.

### Untersuchungen zur Wirkdissoziation an Uterus und Hypophyse

In vivo wird die Wirkung an ERbeta durch eine Stimulierung der Follikulogenese im Ovar der weiblichen Ratte nachgewiesen. Die Wirkung an ERalpha wird durch eine Erhöhung des Uterusgewichts in ovarektomierten Ratten gemessen.

Die Untersuchungen bezügliche der Wirkung auf Uteruswachstum und Ovulation (indirekter Effekt durch Einfluss auf die Sekretion der Hyphysenhormone) werden in adulten weiblichen Ratten (Körpergewicht 220- 250 g) durchgeführt. Die Substanzen werden subkutan einmal pro Tag über 4 Tage appliziert. Die erste Applikation erfolgt im Zyklusstadium Metoestrus. Einen Tag nach der letzten Applikation erfolgt die Autopsie. Erfasst und ausgewertet wird die Anzahl der Oozyten in jedem Eileiter (Effekt auf die Ovulation), ebenso das Uterusfeuchtgewicht.
Während Estradiol eine dosis-abhängige Ovulationsinhibition und eine Zunahme des Uterusgewichts bei einer ED₅₀ von 0,004 mg/kg Körpergewicht bewirkt, zeigt die untersuchte Substanz keinen Effekt auf die Hypophyse und den Uterus.

### Untersuchungen zur Wirkung am Ovar:

Die Substanzen werden in vivo an hypophysektomierten Ratten getestet. In einer Modifikation dieser Operationsmethode wird den Tieren stattdessen ein GnRH-Antagonist (Cetrorelix) verabreicht. Es wird untersucht, ob die Substanz das Follikelwachstum (Maturation) im Ovar stimuliert. Erfasst und ausgewertet wird das Ovargewicht. Pro Behandlungsgruppe werden fünf Tiere (Körpergewicht 40 - 50 g, Alter 3 Wochen) randomisiert eingesetzt. Die Tiere erhalten ad libitum angesäuertes Leitungswasser und eine Standarddiät und werden in Makrolonkäfigen in klimatisierten, kontrolliert beleuchteten Räumen (12 h Licht, 12 h Dunkelheit) gehalten.
Für eine subkutane Applikation werden die Prüfsubstanzen wie auch die Referenzsubstanz (E2, Estradiol) in Benzylbenzoat/Rhizinusöl (1 + 4, v/v) gelöst.

Juvenile weibliche Ratten werden an Tag 0 hypophysektomiert und 1x täglich über 4 Tage subkutan entweder mit der Referenzsubstanz Estradiol, der Prüfsubstanz oder der Trägerlösung (Benzylbenzoat/Rhizinusöl) behandelt. Bei der modifizierten Methode werden 0.5 mg/Tier/Tag Cetrorelix zusammen mit den Prüfsubstanzen bzw. Estradiol oder Trägerlösung über 4 Tage appliziert. 24 Stunden nach der letzten Applikation werden die Tiere getötet und sowohl das Ovargewicht als auch nach histologischer Aufarbeitung die unterschiedlichen Follikeltypen erfasst. Die Prüfsubstanze zeigt eine deutliche Stimulation des Ovargewichts und Erhöhung der Anzahl preantraler Follikel (pro-fertiler Effekt).
Die Prüfsubstanz zeigt eine deutliche Dissoziation zwischen der Wirkung am Ovar selbst und der Wirkung an Uterus und Hypophyse und ist damit aufgrund ihrer profertilen, Follikel-stimulierenden Wirkung explizit für die Behandlung weiblicher Infertilität geeignet.

### Herstellung der erfindungsgemäßen Verbindungen

Die erfindungsgemäßen Verbindungen können mit dem oben beschriebenen Syntheseweg hergestellt werden

Die erfindungsgemäßen Carbonsäureester werden in Analogie zu den Estern hergestellt, die sich von natürlichen Steroidwirkstoffen ableiten (siehe z.B. Pharmazeutische Wirkstoffe, Synthesen, Patente, Anwendungen; A. Kleernann, J. Engel', Georg Thieme Verlag Stuttgart 1978. Arzneimittel, Fortschritte 1972 bis 1985; A. Kleemann, E. Lindner, J. Engel (Hrsg.), VCH 1987, S. 773-814).

Die erfindungsgemäßen Verbindungen der allgemeinen Formel I werden wie in den Beispielen beschrieben hergestellt. Durch analoge Vorgehensweise unter Verwendung homologer Reagenzien zu den in den Beispielen beschriebenen Reagenzien lassen sich weitere Verbindungen der allgemeinen Formel I erhalten.

Veretherung und/oder Veresterung freier Hydroxygruppen erfolgt nach dem Fachmann gängigen Methoden.

### Beispiele

### 1. Herstellung der Nonaflate

### 1.1. Nonafluorobutan-1-sulfonsäure-2-methoxy-8,9-dihydro-7H-benzocyclohepten-6-yl-ester

250 mg 2-Methoxy-5,7,8,9-tetrahydro-benzocyclohepten-6-on werden in 4 ml Toluol bei 0°C gelöst. Nach Zugabe von 0.7 ml DBU werden 0.78 ml Nonaflylfluorid zugegeben. Bei RT wird 2h gerührt. Die Reaktionsmischung wird in 40 ml NH₄Cl-Lsg. gegeben. Nach Extraktion mit Essigsäureethylester wird die organische Phase mit Wasser und ges. NaCl-Lsg. gewaschen, über Na₂SO₄ getrocknet, filtriert, eingeengt und im Vakuum getrocknet. Man erhält quantitativ Nonafluorobutan-1-sulfonsäure-2-methoxy-8,9-dihydro-7H-benzocyclohepten-6-yl-ester.
¹H-NMR(CDCl₃) δ in ppm: 1.98 (m, 2H, CH₂), 2.77 (m, 2H, CH₂), 2.83 (m, 2H, CH₂), 3.80 (s, 3H, OCH₃), 6.55 (s, 1 H, -CH=), 6.66 (m, 1 H, CH_{Ar}), 6.72 (m, 1 H, CH_{Ar}), 7.09 (m, 1H, CH_{Ar}).
¹⁹F-NMR(CDCl₃) δ in ppm: -125.78 (m, 2F, CF₂), -120.83 (m, 2F, CF₂), -110.02 (m, 2F, CF₂), -80.58 (t, 3F, CF₃).

### 1.2. 5-Ethyl-2-methoxy-5,7,8,9-tetrahydro-benzocyclohepten-6-on

830 mg 2-Methoxy-5,7,8,9-tetrahydro-benzocyclohepten-6-on werden in 4.5 ml t.-BuOH gelöst. Nach Zugabe von 516 mg KOt.-Bu und 0.35 ml Ethyliodid wird 1 h in einem Mikrowellenreaktor auf 100°C erhitzt. Das Reaktionsgemisch wird eingeengt, der Rückstand mit Wasser aufgenommen und mit Ether extrahiert. Die organische Phase wird mit Wasser neutral gewaschen, über Na₂SO₄ getrocknet und eingeengt. Nach säulenchromatographischer Reinigung über Kieselgel erhält man 5-Ethyl-2-methoxy-5,7,8,9-tetrahydro-benzocyclohepten-6-on.
¹H-NMR(CDCl₃) δ in ppm: 0.92 (t, 3H, CH₃), 1,74 (m, 1 H, CH₂), 1.91 (m, 1 H, CH₂), 2.07 (m, 1 H, CH₂), 2.24 (m, 1 H, CH₂), 2.46-2.72 (m, 2H, CH₂), 2.80-2.99 (m, 2H, CH₂), 3.64 (t, 1 H, CH), 3.80 (s, 3H, OCH₃), 6.69-6.77 (m, 2H, CH_{Ar}), 7.07 (m, 1 H, CH_{Ar}).

### 1.2.1. Nonafluorobutan-1-sulfonsäure-2-methoxy-5-ethyl-8,9-dihydro-7H-benzocyclo-hepten-6-yl-ester

1.12 g 5-Ethyl-2-methoxy-5,7,8,9-tetrahydro-benzocyclohepten-6-on werden in 12 ml Toluol bei 0°C gelöst. Nach Zugabe von 2.75 ml DBU werden 3.0 ml Nonaflylfluorid zugegeben. Bei RT wird 2h gerührt. Die Reaktionsmischung wird in 120 ml NH₄Cl-Lsg. gegeben. Nach Extraktion mit Essigsäureethylester wird die organische Phase mit Wasser und ges. NaCl-Lsg. gewaschen, über Na₂SO₄ getrocknet, filtriert, eingeengt und im Vakuum getrocknet. Man erhält quantitativ Nonafluorobutan-1-sulfonsäure-2-methoxy-5-ethyl-8,9-dihydro-7H-benzocyclohepten-6-yl-ester.
¹H-NMR(CDCl₃) δ in ppm: 0.96 (t, 3H, CH₃), 2.18-2.30 (m, 4H, 2CH₂), 2.61 (q, 2H, CH₂), 2.80 (m, 2H, CH₂), 3.83 (s, 3H, OCH₃), 6.77-6.84 (m, 2H, CH_{Ar}), 7.06 (m, 1 H, CH_{Ar}).
¹⁹F-NMR(CDCl₃) δ in ppm: -125.78 (m, 2F, CF₂), -120.82 (m, 2F, CF₂), -110.67 (m, 2F, CF₂), -80.57 (t, 3F, CF₃).

### 1.3. 5-Allyl-2-methoxy-5,7,8,9-tetrahydro-benzocyclohepten-6-on

500 mg 2-Methoxy-5,7,8,9-tetrahydro-benzocyclohepten-6-on werden in 3 ml t.-BuOH gelöst. Nach Zugabe von 310 mg KOt.-Bu und 0.22 ml Allylbromid wird 1 h in einem Mikrowellenreaktor auf 100°C erhitzt. Das Reaktionsgemisch wird eingeengt, der Rückstand mit Wasser aufgenommen und mit Ether extrahiert. Die organische Phase wird mit Wasser neutral gewaschen, über Na₂SO₄ getrocknet und eingeengt. Nach säulenchromatographischer Reinigung über Kieselgel erhält man 5-Allyl-2-methoxy-5,7,8,9-tetrahydro-benzocyclohepten-6-on.
¹H-NMR(CDCl₃) δ in ppm: 1.89 (m, 1 H, CH₂), 2.08 (m, 1 H, CH₂), 2.49-2.55 (m, 2H, CH₂), 2.61-2.70 (m, 1H, CH₂), 2.79-2.87 (m, 1 H, CH₂), 2.92-3.02 (m, 2H, CH₂), 3.79 (s, 3H, OCH₃), 3.84 (t, 1 H, CH), 5.12 (m, 2H, CH=CH₂), 5.77 (m, 1 H, CH=CH₂), 6.69-6.77 (m, 2H, CH_{Ar}), 7.06 (m, 1 H, CH_{Ar}).

### 1.3.1. Nonafluorobutan-1-sulfonsäure-2-methoxy-5-allyl-8,9-dihydro-7H-benzocyclohepten-6-yl-ester

405 mg 5-Allyl-2-methoxy-5,7,8,9-tetrahydro-benzocyclohepten-6-on werden in 5 ml Toluol bei 0°C gelöst. Nach Zugabe von 0.94 ml DBU werden 1.04 ml Nonaflylfluorid zugegeben. Bei RT wird 2 h gerührt. Die Reaktionsmischung wird in 60 ml NH₄Cl-Lsg. gegeben. Nach Extraktion mit Essigsäureethylester wird die organische Phase mit Wasser, ges. NaCl-Lsg. gewaschen, über Na₂SO₄ getrocknet, filtriert, eingeengt und im Vakuum getrocknet. Man erhält quantitativ Nonafluorobutan-1-sulfonsäure-2-methoxy-5-allyl-8,9-dihydro-7H-benzocyclohepten-6-yl-ester.
¹H-NMR(CDCl₃) δ in ppm: 2.22-2.33 (m, 4H, 2CH₂), 2.67 (m, 2H, CH₂), 3.35 (m, 2H, CH₂), 3.83 (s, 3H, OCH₃), 4.97-5.10 (m, 2H, CH=CH₂), 5.63-5.75 (m, 1 H, CH=CH₂), 6.65-6.84 (m, 2H, CH_{Ar}), 7.25 (m, 1 H, CH_{Ar}).
¹⁹F-NMR(CDCl₃) δ in ppm: -125.77 (m, 2F, CF₂), -120.81 (m, 2F, CF₂), -110.53 (m, 2F, CF₂), -80.57 (t, 3F, CF₃).

### 1.4. 5,5-Diethyl-2-methoxy-5,7,8,9-tetrahydro-benzocyclohepten-6-on

300 mg 2-Methoxy-5,7,8,9-tetrahydro-benzocyclohepten-6-on werden in 3 ml t.-BuOH gelöst. Nach Zugabe von 375 mg KOt.-Bu und 0.26 ml Ethyliodid wird 1 h in einem Mikrowellenreaktor auf 100°C erhitzt. Das Reaktionsgemisch wird eingeengt, der Rückstand mit Wasser aufgenommen und mit Ether extrahiert. Die organische Phase wird mit Wasser neutral gewaschen, über Na₂SO₄ getrocknet und eingeengt. Nach säulenchromatographischer Reinigung über Kieselgel erhält man 5,5-Diethyl-2-methoxy-5,7,8,9-tetrahydro-benzocyclohepten-6-on.
¹H-NMR(CDCl₃) δ in ppm: 0.71 (m, 6H, 2CH₃), 1,77-2.05 (m, 6H, 3CH₂), 2.44 (m, 2H, CH₂), 2.67 (m, 2H, CH₂), 3.82 (s, 3H, OCH₃), 6.70 (m, 1 H, CH_{Ar}), 6.80 (m, 1 H, CH_{Ar}), 7.18 (m, 1 H, CH_{Ar}).

### 1.4.1. Nonafluorobutan-1-sulfonsäure-2-methoxy-5,5-diethyl-8,9-dihydro-7H-benzocyclohepten-6-yl-ester

145 mg 5,5-Diethyl-2-methoxy-5,7,8,9-tetrahydro-benzocyclohepten-6-on werden in 5 ml THF gelöst. Unter Argon werden bei 0°C 0.44 ml 2M LDA-Lsg. zugegeben. Nach 15 min rühren bei 0°C werden 0.16 ml Nonaflylfluorid zugetropft. Anschließend läßt man 3 h bei RT rühren. Die Reaktionsmischung wird in 20 ml NH₄Cl-Lsg. gegeben. Nach Extraktion mit Essigsäureethylester wird die organische Phase mit Wasser und ges. NaCl-Lsg. gewaschen, über Na₂SO₄ getrocknet, filtriert, eingeengt und im Vakuum getrocknet. Man erhält Nonafluorobutan-1-sulfonsäure-2-methoxy-5,5-diethyl-8,9-dihydro-7H-benzocyclohepten-6-yl-ester.
¹⁹F-NMR(CDCl₃) δ in ppm: -125.9 (m, 2F, CF₂), -121.6 (m, 2F, CF₂), -114.4 (m, 2F, CF₂), -80.8 (t, 3F, CF₃).

### 1.5. Nonafluorobutan-1-sulfonsäure-2-methoxy-6-(4-methoxyphenyl)-8,9-dihydro-7H-benzocyclohepten-5-yl-ester

1.0 g 2-Methoxy-6-(4-methoxyphenyl)-6,7,8,9-tetrahydro-benzocyclohepten-5-on wird in 15 ml Toluol gelöst. Bei 0°C werden 1.8 ml DBU und 2 ml Nonaflylfluorid zugegeben. Nach 3 h Rühren bei RT wird die Reaktionsmischung in 40 ml NH₄Cl-Lsg. gegeben, das Produkt mit Essigsäureethylester extrahiert, die org. Phase mit Wasser und ges. NaCl-Lsg. gewaschen. Über Na₂SO₄ wird getrocknet anschließend filtriert und eingeengt. Man erhält Nonafluorobutan-1-sulfonsäure-2-methoxy-6-(4-methoxyphenyl)-8,9-dihydro-7H-benzocyclohepten-5-yl-ester.
¹H-NMR(CDCl₃) δ in ppm: 2.25 (m, 2H, CH₂), 2.39 (m, 2H, CH₂), 2.85 (m, 2H, CH₂), 3.84 (s, 3H, OCH₃), 3.86 (s, 3H, OCH₃), 6.70-6.98 (m, 4H, CH_{Ar}), 7.37-7.46 (m, 3H, CH_{Ar}).

### 2. Herstellung der Testverbindungen

Variante A
3-Methoxy-8-(4-methoxyphenyl)-9-vinyl-6,7-dihydro-5H-benzocyclohepten
Zu einer Mischung aus 425 mg Nonafluorobutan-1-sulfonsäure-2-methoxy-6-(4-methoxyphenyl)-8,9-dihydro-7H-benzocyclohepten-5-yl-ester, 240 mg CsF, Pd(PPh₃)₄ in 2 ml Dioxan gibt man unter Argon 0.63 ml 10%ige t.-Bu₃P in n-Hexan und 0.25 ml Triethylvinylzinn. Die Reaktionsmischung wird 2 h im Mikrowellenreaktor auf 120°C erhitzt. Die Reaktionsmischung wird über Celite filtriert, eingeengt und das Produkt säulenchromatographisch gereinigt. Man erhält 3-Methoxy-8-(4-methoxyphenyl)-9-vinyl-6,7-dihydro-5H-benzocyclohepten.
¹H-NMR(CDCl₃) δ in ppm: 2.10 (m, 2H, CH₂), 2.35 (m, 2H, CH₂), 2.67 (m, 2H, CH₂), 3.88(s, 6H, 2OCH₃), 4.98-5.09 (m, 2H, CH=CH₂), 6.62-6.73 (m, 1H, CH=CH2), 6.78-6.98 (m, 4H, CH_{Ar}), 7.24-7.33 (m, 3H, CH_{Ar}).
Variante B
6-(4-Hydroxy-Phenyl)-5-vinyl-8,9-dihydro-7H-benzocyclohepten-2-ol
100 mg 3-Methoxy-8-(4-methoxyphenyl)-9-vinyl-6,7-dihydro-5H-benzocyclohepten werden in 4.5 ml CH₂Cl₂ bei -10°C vorgelegt. Es werden 3 ml 1M BBr₃-Lsg. in CH₂Cl₂ zugetropft und anschließend 1 h bei RT gerührt. Die Reaktionsmischung wird in 40 ml Wasser gegeben und mit Essigsäureethylester extrahiert. Die org. Phase wird mit ges. NaCl-Lsg. gewaschen, über Na₂SO₄ getrocknet, filtriert und eingeengt. Nach säulenchromatographischer Reinigung erhält man 3-Hydroxy-8-(4-hydroxyphenyl)-9-vinyl-6,7-dihydro-5H-benzocyclohepten: C₁₉H₁₈O₂: M⁺ = 278 m/e.
¹H-NMR(DMSO-d₆) δ in ppm: 9.19 (s, 1 H, OH), 9.32 (s, 1 H, OH).
Variante C
6-(4-Hydroxy-Phenyl)-8,9-dihydro-7H-benzocyclohepten-2-ol
150 mg Nonafluorobutan-1-sulfonsäure-2-methoxy-8,9-dihydro-7H-benzocyclohepten-6-yl-ester und 70 mg p-Methoxyphenylboronsäure werden in 1.5 ml Toluol und 1.5 ml Ethanol unter Argon vorgelegt. Es werden 22 mg Pd(PPh₃)₄ und 0.61 ml einer 1 M Na₂CO₃-Lsg. zugegeben. Anschließend wird im Mikrowellenreaktor 15 min auf 120°C erhitzt. Die Reaktionsmischung wird eingeengt in 4 ml CH₂Cl₂ aufgenommen. Bei 0°C werden 3 ml 1 M BBr₃-Lsg. zugegeben und bei RT 2 h gerührt. Die Reaktionsmischung wird in 40 ml Wasser gegeben und mit Essigsäureethylester extrahiert. Die org. Phase wird mit ges. NaCl-Lsg. gewaschen, über Na₂SO₄ getrocknet, filtriert und eingeengt. Nach säulenchromatographischer Reinigung erhält man 6-(4-Hydroxyphenyl)-8,9-dihydro-7H-benzocyclohepten-2-ol. ¹H-NMR(DMSO-d₆)_{.} δ in ppm: 2.03 (m, 2H, CH₂), 2.53 (m, 2H, CH₂), 2.65 (m, 2H, CH₂), 6.48-6.61 (m, 3H, CH_{Ar}), 6.74 (m, 2H, CH_{Ar}), 7.04 (m, 1 H, CH_{Ar}), 7.32 (m, 2H, CH_{Ar}), 9.32 (s, 1 H, OH), 9.38 (s, 1 H, OH).
Variante D
5-Ethyl-6-(4-hydroxy-phenyl)-8,9-dihydro-7H-benzocyclohepten-2-ol
20 mg 6-(4-Hydroxy-phenyl)-5-vinyl-8,9-dihydro-7H-benzocyclohepten-2-ol und 10 mg Pd/C (5%) werden in 10 ml Methanol gegeben. Bei RT wird unter Normaldruck hydriert. Die Lösung wird filtriert und eingeengt. Man erhält 5-Ethyl-6-(4-hydroxyphenyl)-8,9-dihydro-7H-benzocyclohepten-2-ol: C₁₉H₂₀O₂: M⁺ = 280 m/e.
Variante E
6-(4-Hydroxy-phenyl)-5-propyl-6,7,8,9-tetrahydro-5H-benzocyclohepten-2-ol 20 mg 5-Allyl-6-(4-hydroxy-phenyl)-8,9-dihydro-7H-benzocyclohepten-2-ol und 10 mg Pd/C (5%) werden in 10 ml Methanol gegeben. Bei RT und 7.5 bar wird hydriert. Die Lösung wird filtriert und eingeengt. Man erhält 6-(4-Hydroxy-phenyl)-5-propyl-6,7,8,9-tetrahydro-5H-benzocyclohepten-2-ol: C₂₀H₂₄O₂: M⁺ = 296 m/e.
Variante F
9-Allyl-3-methoxy-8-(4-methoxyphenyl)-6,7-dihydro-5H-benzocyclohepten 400 mg Nonafluorobutan-1-sulfonsäure-2-methoxy-5-allyl-8,9-dihydro-7H-benzocyclohepten-6-yl-ester und 170 mg p-Methoxyphenylboronsäure werden in 3.7 ml Toluol und 3.7 ml Ethanol unter Argon vorgelegt. Es werden 55 mg Pd(PPh₃)₄ und 1.5 ml einer 1 M Na₂CO₃-Lsg. zugegeben. Anschließend wird im Mikrowellenreaktor 15 min auf 120°C erhitzt. Die Reaktionsmischung wird eingeengt und das Produkt chromatographiert. Man erhält 9-Allyl-3-methoxy-8-(4-methoxyphenyl)-6,7-dihydro-5H-benzocyclohepten.
¹H-NMR(CDCl₃) δ in ppm: 2.07-2.20 (m, 4H, 2CH₂), 2.67 (m, 2H, CH₂), 3.20 (m, 2H, CH₂), 3.84 (s, 6H, 20CH₃), 4.88 (m, 2H, CH=CH₂), 5.67 (m, 1 H, CH=CH2), 6.75-6.83 (m, 4H, CH_{Ar}), 7.22-7.30 (m, 3H, CH_{Ar}).
Variante G
9-Ethyl-3-methoxy-8-(4-methoxyphenyl)-6,7-dihydro-5H-benzocyclohepten 400 mg Nonafluorobutan-1-sulfonsäure-2-methoxy-5-ethyl-8,9-dihydro-7H-benzocyclohepten-6-yl-ester und 170 mg p-Methoxyphenylboronsäure werden in 3.7 ml Toluol und 3.7 ml Ethanol unter Argon vorgelegt. Es werden 55 mg Pd(PPh₃)₄ und 1.5 ml einer 1 M Na₂CO₃-Lsg. zugegeben. Anschließend wird bei RT bis zur vollständigen Umsetzung gerührt. Die Reaktionsmischung wird eingeengt und das Produkt chromatographiert. Man erhält 9-Ethyl-3-methoxy-8-(4-methoxyphenyl)-6,7-dihydro-5H-benzocyclohepten.
¹H-NMR(CDCl₃)_{.} δ in ppm: 0.83 (t, 3H, CH₃), 2.06-2.14 (m, 4H, 2CH₂), 2.43 (q, 2H, CH₂), 2.65 (m, 2H, CH₂), 3.19 (m, 2H, CH₂), 3.78-3.84 (2s+m, 8H, CH₂+2OCH₃), 6.76-6.93 (m, 4H, CH_{Ar}), 7.17-7.25 (m, 3H, CH_{Ar}).
Variante H
5-Ethyl-6-(4-hydroxy-phenyl)-8,9-dihydro-7H-benzocyclohepten-2-ol
50 mg 9-Ethyl-3-methoxy-8-(4-methoxyphenyl)-6,7-dihydro-5H-benzocyclohepten werden in 3.5 ml CH₂Cl₂ gelöst. Nach Zugabe von 230 mg Tetrabutylammoniumiodid wird auf -60°C gekühlt und 1.25 ml 1 M BCl₃-Lsg. in CH₂Cl₂ zugegeben. Nach 1 h bei 0°C wird eine weitere Stunde bei RT gerührt. Anschließend wird mit 10 ml ges. NH4Cl-Lsg. versetzt, nach Phasentrennung zur Trockene eingeengt und säulenchromatographisch gereinigt. Man erhält 6-(4-Hydroxyphenyl)-5-ethyl-6,7,8,9-tetrahydro-5H-benzocyclohepten-2-ol: C₁₉H₂₀O₂, M⁺= 280 m/e.
Variante I
6-(4-Hydroxy-phenyl)-5-propyl-8,9-dihydro-7H-benzocyclohepten-2-ol
60 mg 9-Allyl-3-methoxy-8-(4-methoxyphenyl)-6,7-dihydro-5H-benzocyclohepten werden in 4 ml Toluol unter Argon vorgelegt. Anschließend werden 1.5 ml einer 1.5 M DIBAH-Lsg. in Toluol zugegeben. Die Reaktionsmischung wird 2h unter Rückfluß gekocht. Die erkaltete Reaktionsmischung wird auf 30 ml Eiswasser gegeben. Nach Extraktion mit Essigsäureethylester wird über Na₂SO₄ getrocknet, filtriert und eingeengt. Nach säulenchromatographischer Reinigung erhält man 6-(4-Hydroxyphenyl)-5-propyl-8,9-dihydro-7H-benzocyclohepten-2-ol: C₂₀H₂₂O₂, M⁺= 294 m/e. ¹H-NMR(DMSO-d₆). δ in ppm: 0.67 (t, 3H, CH₃), 1.17 (m, 2H, CH₂), 1.80-2.22 (m(überlg.), 4H, 2CH₂), 2.31 (m, 2H, CH₂), 2.51 (m(überlg.), 2H, CH₂), 6.60-6.78 (m, 4H, CH_{Ar}), 7.00 - 7.13 (m, 3H, CH_{Ar}), 9.22 - 9.38 (2s (br.), 2H, 20H).
Variante J
2-Methoxy-6-(4-methoxyphenyl)-6-allyl-6,7,8,9-tetrahydrobenzocyclohepten-5-on 200 mg 2-Methoxy-6-(4-methoxyphenyl)-6,7,8,9-tetrahydro-benzocyclohepten-5-on werden in 3 ml tert.-Butanol und 1.8 ml Allylbromid gelöst. Nach Zugabe von 265 mg KOt.Bu wird 3 h bei RT gerührt. Nach beendeter Reaktion wird mit 15 ml Wasser verdünnt. Nach Phasentrennung wird mit Essigsäureethylester nachextrahiert, die vereinigten org. Phasen mit NaCl-Lsg. gewaschen, über Na₂SO₄ getrocknet, filtriert und eingeengt.
Nach säulenchromatischer Reinigung an Kieselgel erhält man 2-Methoxy-6-(4-methoxyphenyl)-6-allyl-6,7,8,9-tetrahydrobenzocyclohepten-5-on: C₂₂H₂₄O₃, M⁺= 336 m/e.
¹H-NMR(CDCl₃) δ in ppm: 1.80 (m, 2H, CH₂), 2.05 (m, 1 H, CH₂), 2.20 (m, 1 H, CH₂), 2.39-2.59 (m, 3H, CH₂), 2.92 (m, 2H, CH₂), 3.80 (s, 3H, OCH₃), 3.83 (s, 3H, OCH₃), 4.95-5.07 (m, 2H, =CH₂), 5.60 (m, H, =CH), 6.57 (m, 1 H, CH_{Ar}), 6.80 (m, 1 H, CH_{Ar}), 6.86 (m, 2H, CH_{Ar}), 7.24 (m, 2H, CH_{Ar}), 7.57 (m, 1 H, CH_{Ar}).
Variante K
2-Methoxy-6-(4-methoxy-phenyl)-6-propyl-6,7,8,9-tetrahydro-5H-benzocyclohepten 180 mg 2-Methoxy-6-(4-methoxy-phenyl)-6-propyl-6,7,8,9-tetrahydrobenzocyclohepten-5-on wird unter Argon in 15 ml CH₂Cl₂ und 8 ml Triethylsilan vorgelegt. Bei -40°C werden 15 ml Bortrifluoridetherat zugetropft. Anschließend wird 3 h bei RT gerührt. Unter Kühlung werden langsam 40 ml 10%ige K₂CO₃-Lsg. zugegeben. Nach 8 h rühren bei RT werden die Phasen getrennt, mit Essigsäureethylester nachextrahiert, die vereinigten org. Phasen mit Wasser und ges. NaCl-Lsg. gewaschen. Nach Trocknung über Na₂SO₄ wird filtriert und eingeengt. Man erhält 2-Methoxy-6-(4-methoxy-phenyl)-6-propyl-6,7,8,9-tetrahydro-5H-benzocyclohepten: C₂₂H₂₈O₂, M⁺= 324 m/e.
¹H-NMR(CDCl₃) δ in ppm: 0.70 (t, 3H, CH₃), 0.98 (m, 2H, CH₂), 1.41 (m, 2H, CH₂), 1.75 (m, 2H, CH₂), 1.84-2.13 (m, 2H, CH₂), 2.75 (m, 2H, CH₂), 2.95-3.21 (m, 2H, CH₂), 3.78-3.84 (2s, 6H, OCH₃), 6.56-6.66 (m, 3H, CH_{Ar}), 6.85 (m, 2H, CH_{Ar}), 7.02 (m, 1 H, CH_{Ar}), 7.30 (m, 2H, CH_{Ar}).

| Beispiel | Herstellung (Variante) | Edukte | Strukturformel | Gefundene Molmasse |
|---|---|---|---|---|
| 1 | A+B | 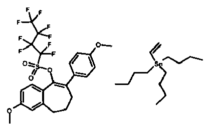 | 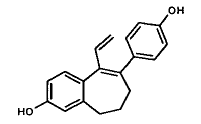 | 278 |
| 2 | C | 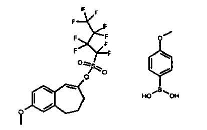 | 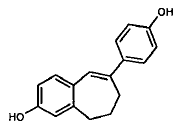 | 252 |
| 3 | D | 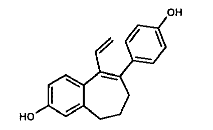 | 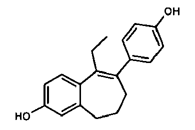 | 280 |
| 3 | G+H | 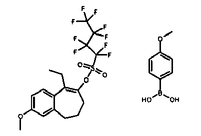 | 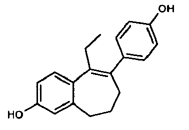 | 280 |
| 3 | A+H | 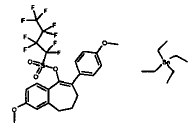 | 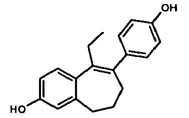 | 280 |
| 4 | F+H | 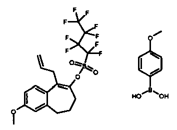 | 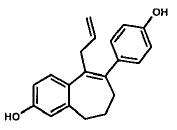 | 292 |
| 5 | E | 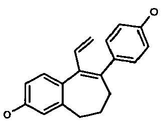 | 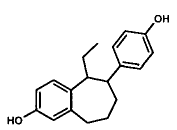 | 282 |
| 6 | I | 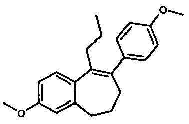 | 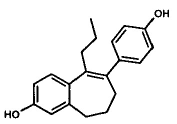 | 294 |
| 6 | D | 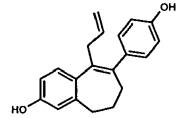 | 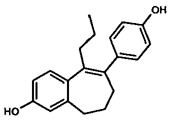 | 294 |
| 7 | E | 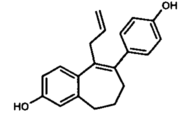 | 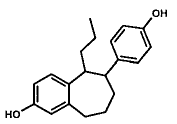 | 296 |
| 8 | G+B | 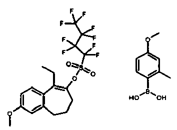 | 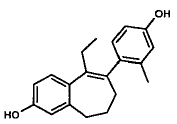 | 294 |
| 9 | J+K+B | 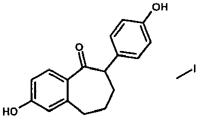 | 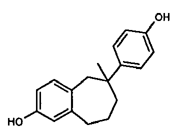 | 268 |
| 10 | J+K+B | 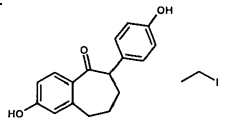 | 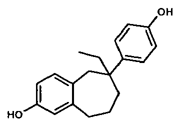 | 282 |
| 11 | J+K+B | 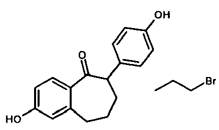 | 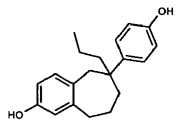 | 296 |
| 12 | J+K+B | 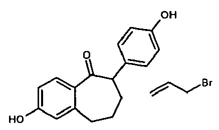 | 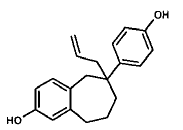 | 294 |
| 13 | A + I | 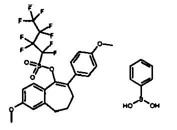 | 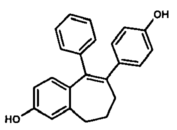 | 328 |
| 14 | A + I | 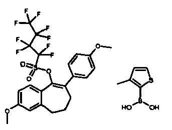 | 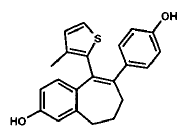 | 348 |
| 15 | F + I | 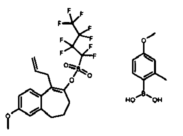 | 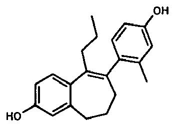 | 308 |
| 16 | F + I | 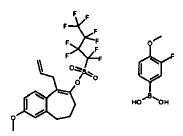 | 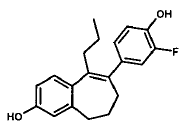 | 312 |
| 17 | F + I | 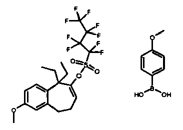 | 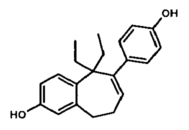 | 308 |
| 18 | C | 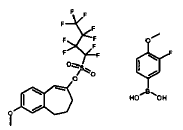 | 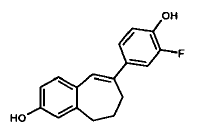 | 270 |
| 19 | D | 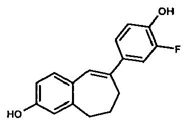 | 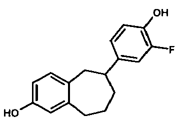 | 272 |
| 20 | G+D | 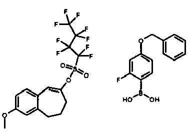 | 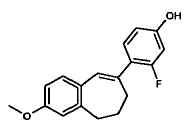 | 284 |
| 21 | G+D+B | 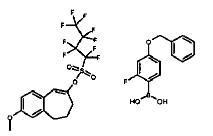 | 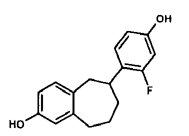 | 272 |

**Tabelle 1**

| Estrogen | Struktur | hER α RBA* | hER β RBA* | ERβ/ ERα | Rat uterus ER(RBA) | Rat prost. ER(RBA) | prost.ER/ uterusER |
|---|---|---|---|---|---|---|---|
| Estradiol | 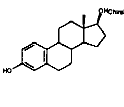 | 100 | 100 | 1 | 100 | 100 | 1 |
| Estron | 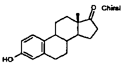 | 60 | 37 | 0.6 | 3 | 2 | 0.8 |
| 17α-Estradiol | 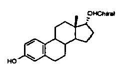 | 58 | 11 | 0.2 | 2.4 | 1.3 | 0.5 |
| Estriol | 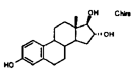 | 14 | 21 | 1.5 | 4 | 20 | 5 |
| 5-Androstendiol | 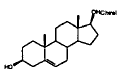 | 6 | 17 | 3 | 0.1 | 5 | 50 |
| Genistein | 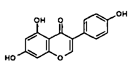 | 5 | 36 | 7 | 0.1 | 10 | 100 |
| Coumestrol | 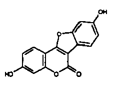 | 94 | 185 | 2 | 1.3 | 24 | 18 |

| | | | | | | | |
|---|---|---|---|---|---|---|---|
| *: zitiert aus : Kuiper et al. (1996), Endocrinology 138: 863-870 | | | | | | | |

**Tabelle 2**

| Structure | Rat Uterus ER(RBA) | Rat Prostate ER(RBA) | Prostate ER / Uterus ER |
|---|---|---|---|
| 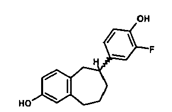 | 1,5 | 1,3 | 0,9 |
| 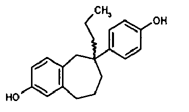 | 2 | 0,7 | 0,4 |
| 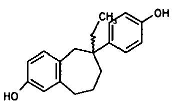 | 3 | 5 | 1,7 |
| 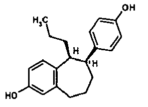 | 30 | 25 | 0,8 |
| 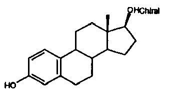 | 100 | 100 | 1 |

**Tabelle 3**

| MOLSTRUCTURE | | | ERa EC50 (GAL4-Assay) | | Potency at ERa (% of E2) | ERb EC50 (GAL4-Assay) | | Potency at ERb (% of E2) |
|---|---|---|---|---|---|---|---|---|
| 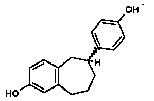 | | > | 1,00E-05 | < | 0,10 | 8,98E-10 | | 8,03 |
| 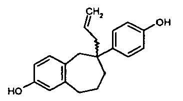 | | > | 1,00E-05 | < | 0,10 | 1,47E-08 | | 0,49 |
| 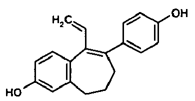 | | | 3,39E-08 | | 0,63 | 1,43E-09 | | 5,04 |
| 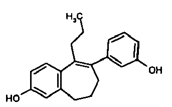 | | | 6,78E-08 | | 0,32 | 4,51E-10 | | 15,99 |
| 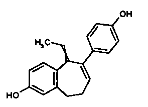 | | | 5,91 E-07 | < | 0,10 | 9,99E-09 | | 0,72 |
| 17b-Estradiol | | | 2,14E-10 | | 100,00 | 7,21E-11 | | 100,00 |

## Patentansprüche

1. Verbindungen der allgemeinen Formel (I) 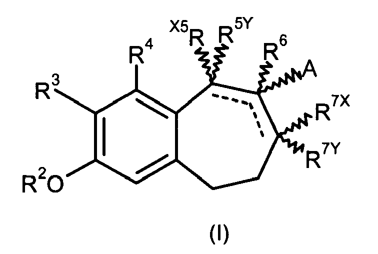 worin
A 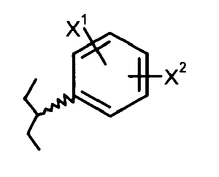 bedeutet worin
X¹ ein oder mehrere Gruppen am Phenylring bedeutet und unabhängig von einander ein Halogen, OH, (C₁-C₄)Alkyl-, (C₁-C₄)Alkyl-O-, (C₃-C₆)Cyclo-alkyl-O, (C₁-C₁₄)Acyl-O-, (C₁-C₄)Alkenyl-, (C₁-C₄)Alkinyl-, Perfluor(C₁-C₄)Alkylrest, -CHO oder CN darstellt, und
X² ein oder mehrere Gruppen am Phenylring bedeutet und unabhängig von einander ein H, Halogen, OH, (C₁-C₄)Alkyl-, (C₁-C₄)Alkyl-O-, (C₁-C₄)Alkenyl-, (C₁-C₄)Alkinyl-, Perfluor(C₁-C₄)Alkylrest, -CHO oder CN darstellt;
R² ein H-Atom, ein (C₁-C₄)Alkylrest, ein (C₃-C₆)Cyclo-alkyl-rest oder ein (C₁-C₁₄)Acylrest ist;
R³ ein H- oder ein F-Atom ist;
R⁴ ein H-Atom, ein (C₁-C₄)Alkyl-, (C₁-C₄)Alkenyl-, (C₁-C₄)Alkinyl-, Perfluor(C₁-C₄)alkyl-, Silyl(C₁-C₄)alkylrest, ein Halogenatom oder ein Nitrilrest ist;
R^{5X}und R^{5Y} unabhängig von einander ein H-Atom, einen (C₁-C₄)Alkyl-, (C₁-C₄)Alkenyl-, (C₁-C₄)Alkinyl-, Perfluor(C₁-C₄)alkyl-, Perfluor(C₁-C₄)alkenyl-, (C₁-C₄)Alkyl-O-, Aryl-, Heteroaryl- oder eine CN-Gruppe bedeuten, oder
R^{5X}und R^{5Y} gemeinsam ein O-, ein S-Atom, ein =CHZ worin Z ein H-Atom, ein (C₁-C₄)Alkyl-, (C₁-C₄)Alkenylrest bedeutet, oder
R^{5X}und R^{5Y} gemeinsam ein -(C₂-C₄)Alkandiyl-, (C₂-C₆)Alkendiylrest oder -OCH₂- sind, oder
R^{5Y}und R⁶ gemeinsam eine Bindung, ein -(C₁-C₄)Alkandiyl- oder einen (C₂-C₆)Alkendiylrest darstellen, und R^{5X} hat die oben gegebenen Bedeutung;
R⁶ ein H-Atom, ein (C₁-C₄)Alkyl-, ein (C₁-C₄)Alkenyl-, (C₁-C₄)Alkinyl-, ein Perfluor(C₁-C₄)alkyl- oder ein Perfluor(C₁-C₄)alkenylrest bedeutet, oder
R⁶und R^{5Y} gemeinsam eine Bindung, ein -(C₁-C₄)Alkandiyl- oder einen (C₂-C₆)Alkendiylrest darstellen, und R^{5X} hat die oben gegebenen Bedeutung oder
R⁶und R^{7Y} gemeinsam eine Bindung, ein -(C₁-C₄)Alkandiyl- oder einen (C₂-C₆)Alkendiylrest darstellen, und R^{7X} hat die unter gegebenen Bedeutung;
R^{7x}und R^{7Y} unabhängig von einander ein H-Atom, ein (C₁-C₄)-Alkyl-, ein (C₁-C₄)Alkenylrest, ein (C₁-C₄)Alkinyl- darstellen, oder
R^{7x}und R^{7Y} gemeinsam ein -(C₂-C₄)Alkandiyl-, oder (C₂-C₆)Alkendiylrest darstellen, oder
R^{7Y}und R⁶ gemeinsam eine Bindung, ein -(C₁-C₄)Alkandiyl- oder einen (C₂-C₆)Alkendiylrest darstellen, und R^{7X} hat die oben gegebenen Bedeutung.

2. Verbindungen der allgemeinen Formel (I) nach Anspruch 1 worin
A 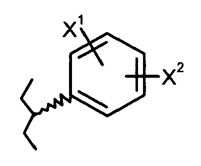 bedeutet worin
X¹ ein oder mehrere Gruppen am Phenylring bedeutet und unabhängig von einander ein Halogen, OH, (C₁-C₄)Alkyl-, (C₁-C₄)Alkyl-O-, (C₃-C₆)Cyclo-alkyl-O, (C₁-C₁₄)Acyl-O-, (C₁-C₄)Alkenyl-, (C₁-C₄)Alkinyl-, Perfluor(C₁-C₄)Alkylrest, -CHO oder CN darstellt, und
X² ein oder mehrere Gruppen am Phenylring bedeutet und unabhängig von einander ein H, Halogen, OH, (C₁-C₄)Alkyl-, (C₁-C₄)Alkyl-O-, (C₁-C₄)Alkenyl-, (C₁-C₄)Alkinyl-, Perfluor(C₁-C₄)Alkylrest, -CHO oder CN darstellt;
R² ein H-Atom, ein (C₁-C₄)Alkylrest, ein (C₃-C₆)Cyclo-alkyl-rest oder ein (C₁-C₁₄)Acylrest ist;
R³ ein H- oder ein F-Atom ist;
R⁴ ein H-Atom, ein (C₁-C₄)Alkyl-, (C₁-C₄)Alkenyl-, (C₁-C₄)Alkinyl-, Perfluor(C₁-C₄)alkyl-, Silyl(C₁-C₄)alkylrest, ein Halogenatom oder ein Nitrilrest ist;
R^{5X} ein H-Atom, einen (C₁-C₄)Alkyl-, (C₁-C₄)Alkenyl-, (C₁-C₄)Alkinyl-, Perfluor(C₁-C₄)alkyl-, Perfluor(C₁-C₄)alkenyl-, (C₁-C₄)Alkyl-O-, Aryl-, Heteroaryl- oder eine CN-Gruppe bedeuten;
R^{5Y}und R⁶ gemeinsam eine Bindung darstellen;
R^{7X}und R^{7Y} unabhängig von einander ein H-Atom, ein (C₁-C₄)-Alkyl-, ein (C₁-C₄)Alkenylrest, ein (C₁-C₄)Alkinyl- darstellen, oder
R^{7X}und R^{7Y} gemeinsam -(C₂-C₄)Alkandiyl-, oder (C₂-C₆)Alkendiylrest darstellen.

3. Verbindungen der allgemeinen Formel (I) nach Anspruch 2
worin R^{5X} ein H-Atom bedeutet, und
R^{7X} und R^{7Y} unabhängig von einander ein H-Atom, ein (C₁-C₄)-Alkyl-, ein (C₁-C₄)Alkenylrest, ein (C₁-C₄)Alkinyl- bedeuten.

4. Verbindungen der allgemeinen Formel (I) nach Anspruch 2 bis 3
worin R^{5X}, und R^{7Y} ein H-Atom bedeuten, und
R^{7X} ein H-Atom, ein (C₁-C₄)-Alkyl-, ein (C₁-C₄)Alkenylrest, oder ein (C₁-C₄)Alkinyl- darstellt

5. Verbindungen der allgemeinen Formel (I) nach Anspruch 2
worin R^{5X} ein H-Atom bedeutet, und
R^{7X} und R^{7Y} gemeinsam ein -CH₂-CH=CH-CH₂- oder (-CH₂-CH₂-CH₂-CH₂-) darstellen.

6. Verbindungen der allgemeinen Formel (I) nach Anspruch 2
worin R^{5X} einen (C₁-C₄)Alkyl-, (C₁-C₄)Alkenyl-, (C₁-C₄)Alkinyl-, Perfluor(C₁-C₄)alkyl-, Perfluor(C₁-C₄)alkenyl-, (C₁-C₄)Alkyl-O-, Aryl-, Heteroaryl- oder eine CN-Gruppe bedeuten, und
R^{7X} und R^{7Y} unabhängig von einander ein H-Atom, ein (C₁-C₄)-Alkyl-, ein (C₁-C₄)Alkenylrest, oder ein (C₁-C₄)Alkinyl- bedeuten.

7. Verbindungen der allgemeinen Formel (I) nach Anspruch 1
worin
A 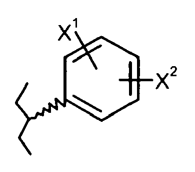 bedeutet worin
X¹ ein oder mehrere Gruppen am Phenylring bedeutet und unabhängig von einander ein Halogen, OH, (C₁-C₄)Alkyl-, (C₁-C₄)Alkyl-O-, (C₃-C₆)Cyclo-alkyl-O, (C₁-C₁₄)Acyl-O-, (C₁-C₄)Alkenyl-, (C₁-C₄)Alkinyl-, Perfluor(C₁-C₄)Alkylrest, -CHO oder CN darstellt, und
X² ein oder mehrere Gruppen am Phenylring bedeutet und unabhängig von einander ein H, Halogen, OH, (C₁-C₄)Alkyl-, (C₁-C₄)Alkyl-O-, (C₁-C₄)Alkenyl-, (C₁-C₄)Alkinyl-, Perfluor(C₁-C₄)Alkylrest, -CHO oder CN darstellt;
R² ein H-Atom, ein (C₁-C₄)Alkylrest, ein (C₃-C₆)Cyclo-alkyl-rest oder ein (C₁-C₁₄)Acylrest ist;
R³ ein H- oder ein F-Atom ist;
R⁴ ein H-Atom, ein (C₁-C₄)Alkyl-, (C₁-C₄)Alkenyl-, (C₁-C₄)Alkinyl-, Perfluor(C₁-C₄)alkyl-, Silyl(C₁-C₄)alkylrest, ein Halogenatom oder ein Nitrilrest ist;
R^{5X}und R^{5Y} unabhängig von einander ein H-Atom, einen (C₁-C₄)Alkyl-, (C₁-C₄)Alkenyl-, (C₁-C₄)Alkinyl-, Perfluor(C₁-C₄)alkyl-, Perfluor(C₁-C₄)alkenyl-, (C₁-C₄)Alkyl-O-, Aryl-, Heteroaryl- oder eine CN-Gruppe bedeuten, oder
R^{5X}und R^{5Y} gemeinsam ein O-, ein S-Atom, ein =CHZ worin Z ein H-Atom, ein (C₁-C₄)Alkyl-, (C₁-C₄)Alkenylrest bedeutet, oder
R^{5X}und R^{5Y} gemeinsam ein -(C₂-C₄)Alkandiyl-, (C₂-C₆)Alkendiylrest oder -OCH₂- sind;
R⁶und R^{7Y} gemeinsam eine Bindung darstellen;
R^{7X} ein H-Atom, ein (C₁-C₄)-Alkyl-, ein (C₁-C₄)Alkenylrest, ein (C₁-C₄)Alkinyldarstellt.

8. Verbindungen der allgemeinen Formel (I) nach Anspruch 7 worin
R^{5X}und R^{5Y} unabhängig von einander ein H-Atom, einen (C₁-C₄)Alkyl-, (C₁-C₄)Alkenyl-, (C₁-C₄)Alkinyl-, Perfluor(C₁-C₄)alkyl-, Perfluor(C₁-C₄)alkenyl-, (C₁-C₄)Alkyl-O-, Aryl-, Heteroaryl- oder eine CN-Gruppe bedeuten;
R^{7X} ein H-Atom bedeutet.

9. Verbindungen der allgemeinen Formel (I) nach Anspruch 7
worin R^{5X}, und R^{7Y} ein H-Atom bedeuten.

10. Verbindungen der allgemeinen Formel (I) nach Anspruch 7
worin R^{5X}, R^{5y}, und R^{7X} ein H-Atom bedeuten.

11. Verbindungen der allgemeinen Formel (I) nach Anspruch 7
worin R^{5X} ein H-Atom bedeutet, und
R^{7X} und R^{7Y} unabhängig von einander ein (C₁-C₄)-Alkyl-, ein (C₁-C₄)Alkenylrest oder ein (C₁-C₄)Alkinyl- darstellen.

12. Verbindungen der allgemeinen Formel (I) nach Anspruch 7
worin R^{5X}, und R^{7Y} ein H-Atom bedeuten, und
R^{7X} ein H-Atom, ein (C₁-C₄)-Alkyl-, ein (C₁-C₄)Alkenylrest, oder ein (C₁-C₄)Alkinyl- darstellt

13. Verbindungen der allgemeinen Formel (I) nach Anspruch 7
worin R^{5X} ein H-Atom bedeutet, und
R^{7X} und R^{7Y} gemeinsam ein ein -(C₂-C₄)Alkandiyl-, oder (C₂-C₆)Alkendiylrest darstellen.

14. Verbindungen der allgemeinen Formel (I) nach Anspruch 7
worin R^{5X} einen (C₁-C₄)Alkyl-, (C₁-C₄)Alkenyl-, (C₁-C₄)Alkinyl-, Perfluor(C₁-C₄)alkyl-, Perfluor(C₁-C₄)alkenyl-, (C₁-C₄)Alkyl-O-, Aryl-, Heteroaryl- eine CN-Gruppe bedeuten, und
R^{7X} und R^{7Y} unabhängig von einander ein H-Atom, ein (C₁-C₄)-Alkyl-, ein (C₁-C₄)Alkenylrest, oder ein (C₁-C₄)Alkinyl- bedeuten.

15. Verbindungen der allgemeinen Formel (I) nach Anspruch 1
worin
A 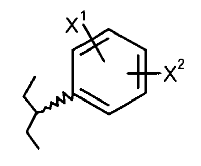 bedeutet worin
X¹ ein oder mehrere Gruppen am Phenylring bedeutet und unabhängig von einander ein Halogen, OH, (C₁-C₄)Alkyl-, (C₁-C₄)Alkyl-O-, (C₃-C₆)Cyclo-alkyl-O, (C₁-C₁₄)Acyl-O-, (C₁-C₄)Alkenyl-, (C₁-C₄)Alkinyl-, Perfluor(C₁-C₄)Alkylrest, -CHO oder CN darstellt, und
X² ein oder mehrere Gruppen am Phenylring bedeutet und unabhängig von einander ein H, Halogen, OH, (C₁-C₄)Alkyl-, (C₁-C₄)Alkyl-O-, (C₁-C₄)Alkenyl-, (C₁-C₄)Alkinyl-, Perfluor(C₁-C₄)Alkylrest, -CHO oder CN darstellt;
R² ein H-Atom, ein (C₁-C₄)Alkylrest, ein (C₃-C₆)Cyclo-alkyl-rest oder ein (C₁-C₁₄)Acylrest ist;
R³ ein H- oder ein F-Atom ist;
R⁴ ein H-Atom, ein (C₁-C₄)Alkyl-, (C₁-C₄)Alkenyl-, (C₁-C₄)Alkinyl-, Perfluor(C₁-C₄)alkyl-, Silyl(C₁-C₄)alkylrest, ein Halogenatom oder ein Nitrilrest ist;
R^{5X}und R^{5Y} unabhängig von einander ein H-Atom, einen (C₁-C₄)Alkyl-,(C₁-C₄)Alkenyl-, (C₁-C₄)Alkinyl-, Perfluor(C₁-C₄)alkyl-, Perfluor(C₁-C₄)alkenyl-, (C₁-C₄)Alkyl-O-, Aryl-, Heteroaryl- oder eine CN-Gruppe bedeuten;
R⁶ ein H-Atom, ein (C₁-C₄)Alkyl-, ein (C₁-C₄)Alkenyl-, (C₁-C₄)Alkinyl-, ein Perfluor(C₁-C₄)alkyl- oder ein Perfluor(C₁-C₄)alkenylrest bedeutet;
R^{7X}und R^{7Y} unabhängig von einander ein H-Atom, ein (C₁-C₄)-Alkyl-, ein (C₁-C₄)Alkenylrest, ein (C₁-C₄)Alkinyl- darstellen.

16. Verbindungen der allgemeinen Formel (I) nach Anspruch 15 worin
R^{5X} ein H-Atom ist;
R^{5Y} ein H-Atom, einen (C₁-C₄)Alkyl-, (C₁-C₄)Alkenyl-, (C₁-C₄)Alkinyl-, Perfluor(C₁-C₄)alkyl-, Perfluor(C₁-C₄)alkenyl-, (C₁-C₄)Alkyl-O-, Aryl-, Heteroaryl- oder eine CN-Gruppe bedeuten;
R⁶ ein H-Atom, ein (C₁-C₄)Alkyl-, ein (C₁-C₄)Alkenyl-, (C₁-C₄)Alkinyl-, ein Perfluor(C₁-C₄)alkyl- oder ein Perfluor(C₁-C₄)alkenylrest bedeutet;
R^{7X}und R^{7Y} unabhängig von einander ein H-Atom, ein (C₁-C₄)-Alkyl-, ein (C₁-C₄)Alkenylrest, ein (C₁-C₄)Alkinyl- darstellen.

17. Verbindungen der allgemeinen Formel (I) nach Anspruch 15 worin
R^{5X}und R^{5Y} ein H-Atom sind;
R⁶ ein H-Atom, ein (C₁-C₄)Alkyl-, ein (C₁-C₄)Alkenyl-, (C₁-C₄)Alkinyl-, ein Perfluor(C₁-C₄)alkyl- oder ein Perfluor(C₁-C₄)alkenylrest bedeutet;
R^{7X}und R^{7Y} unabhängig von einander ein H-Atom, ein (C₁-C₄)-Alkyl-, ein (C₁-C₄)Alkenylrest, ein (C₁-C₄)Alkinyl- darstellen.

18. Verbindungen der allgemeinen Formel (I) nach Anspruch 15 worin
R^{5X}und R^{5Y} unabhängig von einander ein H-Atom, einen (C₁-C₄)Alkyl-, (C₁-C₄)Alkenyl-, (C₁-C₄)Alkinyl-, Perfluor(C₁-C₄)alkyl-, Perfluor(C₁-C₄)alkenyl-, (C₁-C₄)Alkyl-O-, Aryl-, Heteroaryl- oder eine CN-Gruppe bedeuten;
R⁶ ein H-Atom, ein (C₁-C₄)Alkyl-, ein (C₁-C₄)Alkenyl-, (C₁-C₄)Alkinyl-, ein Perfluor(C₁-C₄)alkyl- oder ein Perfluor(C₁-C₄)alkenylrest bedeutet;
R^{7X}und R^{7Y} ein H-Atom sind.

19. Verbindungen der allgemeinen Formel (I) nach Anspruch 15 worin
R^{5X}und R^{5Y} unabhängig von einander ein H-Atom, einen (C₁-C₄)Alkyl-, (C₁-C₄)Alkenyl-, (C₁-C₄)Alkinyl-, Perfluor(C₁-C₄)alkyl-, Perfluor(C₁-C₄)alkenyl-, (C₁-C₄)Alkyl-O-, Aryl-, Heteroaryl- oder eine CN-Gruppe bedeuten;
R⁶ ein H-Atom, ein (C₁-C₄)Alkyl-, ein (C₁-C₄)Alkenyl-, (C₁-C₄)Alkinyl-, ein Perfluor(C₁-C₄)alkyl- oder ein Perfluor(C₁-C₄)alkenylrest bedeutet darstellen;
R^{7X} ein H-Atom ist;
R^{7Y} ein H-Atom, ein (C₁-C₄)-Alkyl-, ein (C₁-C₄)Alkenyl, oder ein (C₁-C₄)Alkinylrest darstellen.

20. Verbindungen der allgemeinen Formel (I) nach Anspruch 15 bis 19 worin R⁶ ein H-Atom ist.

21. Verbindungen der allgemeinen Formel (I) nach Anspruch 1,
worin
A 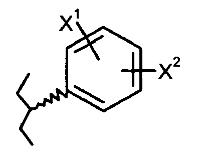 bedeutet worin
X¹ ein oder mehrere Gruppen am Phenylring bedeutet und unabhängig von einander ein Halogen, OH, (C₁-C₄)Alkyl-, (C₁-C₄)Alkyl-O-, (C₃-C₆)Cyclo-alkyl-O, (C₁-C₁₄)Acyl-O-, (C₁-C₄)Alkenyl-, (C₁-C₄)Alkinyl-, Perfluor(C₁-C₄)Alkylrest, -CHO oder CN darstellt, und
X² ein oder mehrere Gruppen am Phenylring bedeutet und unabhängig von einander ein H, Halogen, OH, (C₁-C₄)Alkyl-, (C₁-C₄)Alkyl-O-, (C₁-C₄)Alkenyl-, (C₁-C₄)Alkinyl-, Perfluor(C₁-C₄)Alkylrest, -CHO oder CN darstellt;
R² ein H-Atom, ein (C₁-C₄)Alkylrest, ein (C₃-C₆)Cyclo-alkyl-rest oder ein (C₁-C₁₄)Acylrest ist;
R³ ein H- oder ein F-Atom ist;
R⁴ ein H-Atom, ein (C₁-C₄)Alkyl-, (C₁-C₄)Alkenyl-, (C₁-C₄)Alkinyl-, Perfluor(C₁-C₄)alkyl-, Silyl(C₁-C₄)alkylrest, ein Halogenatom oder ein Nitrilrest ist;
R^{5X}und R^{5Y} gemeinsam ein =O oder =CHZ worin Z ein H-Atom, ein (C₁-C₄)Alkyl-, oder (C₁-C₄)Alkenylrest darstellen;
R⁶ ein H-Atom, ein (C₁-C₄)Alkyl-, ein (C₁-C₄)Alkenyl-, (C₁-C₄)Alkinyl-, ein Perfluor(C₁-C₄)alkyl- oder ein Perfluor(C₁-C₄)alkenylrest bedeutet, oder
R⁶und R^{7Y} gemeinsam eine Bindung, ein -(C₁-C₄)Alkandiyl- oder einen (C₂-C₆)Alkendiylrest darstellen, und R^{7X} hat die unter gegebenen Bedeutung;
R^{7X}und R^{7Y} unabhängig von einander ein H-Atom, ein (C₁-C₄)-Alkyl-, ein (C₁-C₄)Alkenylrest, ein (C₁-C₄)Alkinyl-, oder
R^{7X}und R^{7Y} gemeinsam ein -(C₂-C₄)Alkandiyl-, oder (C₂-C₆)Alkendiylrest darstellen, oder
R^{7Y}und R⁶ gemeinsam eine Bindung, ein -(C₁-C₄)Alkandiyl- oder einen (C₂-C₆)Alkendiylrest darstellen, und R^{7X} hat die oben gegebenen Bedeutung.

22. Verbindungen der allgemeinen Formel (I) nach Anspruch 21 worin
R⁶ ein H-Atom, ein (C₁-C₄)Alkyl-, ein (C₁-C₄)Alkenyl-, (C₁-C₄)Alkinyl-, ein Perfluor(C₁-C₄)alkyl- oder ein Perfluor(C₁-C₄)alkenylrest bedeutet;
R^{7x}und R^{7Y} unabhängig von einander ein H-Atom, ein (C₁-C₄)-Alkyl-, ein (C₁-C₄)Alkenylrest, ein (C₁-C₄)Alkinyl- darstellen.

23. Verbindungen der allgemeinen Formel (I) nach Anspruch 21 worin
R⁶ ein H-Atom, ein (C₁-C₄)Alkyl-, ein (C₁-C₄)Alkenyl-, (C₁-C₄)Alkinyl-, ein Perfluor(C₁-C₄)alkyl- oder ein Perfluor(C₁-C₄)alkenylrest bedeutet;
R^{7x}und R^{7Y} ein H-Atom sind.

24. Verbindungen der allgemeinen Formel (I) nach Anspruch 21 oder 22 worin
R⁶ ein H-Atom, ein (C₁-C₄)Alkyl-, ein (C₁-C₄)Alkenyl-, (C₁-C₄)Alkinyl-, ein Perfluor(C₁-C₄)alkyl- oder ein Perfluor(C₁-C₄)alkenylrest bedeutet;
R^{7X} ein H-Atom ist;
R^{7Y} ein H-Atom, ein (C₁-C₄)-Alkyl-, ein (C₁-C₄)Alkenyl, oder ein (C₁-C₄)Alkinylrest bedeutet.

25. Verbindungen der allgemeinen Formel (I) nach Anspruch 21 bis 24, worin R⁶ ein H-Atom ist.

26. Verbindungen der allgemeinen Formel (I) nach Anspruch 1, 2, 7, 15 oder 20
worin
A 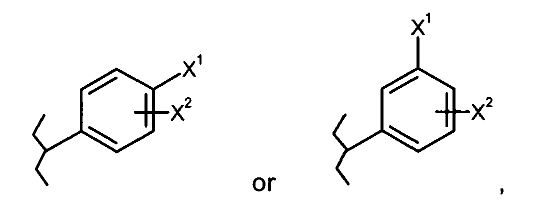 bedeutet worin
X¹ ein Halogen, OH, (C₁-C₄)Alkyl-, (C₁-C₄)Alkyl-O-, (C₃-C₆)Cyclo-alkyl-O, (C₁-C₁₄)Acyl-O-, (C₁-C₄)Alkenyl-, (C₁-C₄)Alkinyl-, Perfluor(C₁-C₄)Alkylrest, -CHO oder CN bedeutet,
X² ein oder mehrere Gruppen am Phenylring bedeutet und unabhängig von einander ein H, Halogen, OH, (C₁-C₄)Alkyl-, (C₁-C₄)Alkyl-O-, (C₁-C₄)Alkenyl-, (C₁-C₄)Alkinyl-, Perfluor(C₁-C₄)Alkylrest, -CHO oder CN darstellt.

27. Verbindungen der allgemeinen Formel (I) nach Anspruch 26,
worin
A 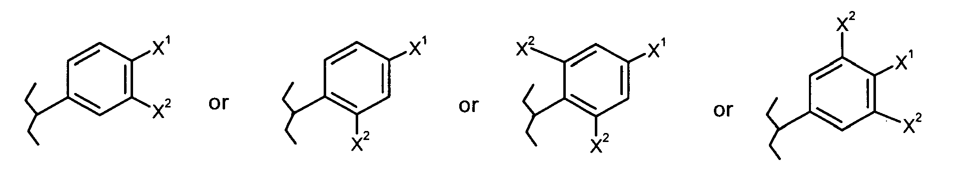 bedeutet, worin
X¹ ein Halogen, OH, (C₁-C₄)Alkyl-, (C₁-C₄)Alkyl-O-, (C₃-C₆)Cyclo-alkyl-O, (C₁-C₁₄)Acyl-O-, (C₁-C₄)Alkenyl-, (C₁-C₄)Alkinyl-, Perfluor(C₁-C₄)Alkylrest, -CHO oder CN bedeutet, und
X² ein H, Halogen, OH, (C₁-C₄)Alkyl-, (C₁-C₄)Alkyl-O-, (C₁-C₄)Alkenyl-, (C₁-C₄)Alkinyl-, Perfluor(C₁-C₄)Alkylrest, -CHO oder CN bedeutet.

28. Verbindungen der allgemeinen Formel (I) nach Anspruch 26,
worin
A 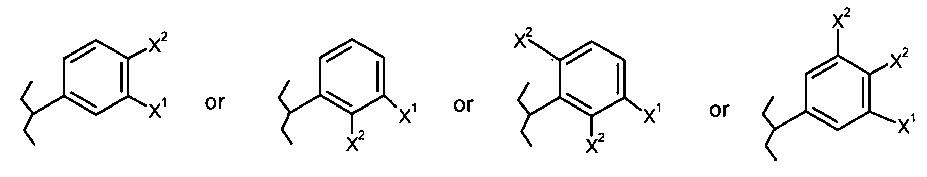 bedeutet, worin
X¹ ein Halogen, OH, (C₁-C₄)Alkyl-, (C₁-C₄)Alkyl-O-, (C₃-C₆)Cyclo-alkyl-O, (C₁-C₁₄)Acyl-O-, (C₁-C₄)Alkenyl-, (C₁-C₄)Alkinyl-, Perfluor(C₁-C₄)Alkylrest, -CHO oder CN bedeutet, und
X², ein H, Halogen, OH, (C₁-C₄)Alkyl-, (C₁-C₄)Alkyl-O-, (C₁-C₄)Alkenyl-, (C₁-C₄)Alkinyl-, Perfluor(C₁-C₄)Alkylrest, -CHO oder CN bedeutet.

29. Verbindungen der allgemeinen Formel (I) nach Anspruch 26 bis 28,
worin
X¹ ein OH, (C₁-C₄)Alkyl-O- oder (C₃-C₆)Cyclo-alkyl-O, bedeutet

30. Verbindungen der allgemeinen Formel (I) nach Anspruch 26 bis 28,
worin
X², ein Halogen, OH, (C₁-C₄)Alkyl-, oder (C₁-C₄)Alkyl-O- bedeutet.

31. Verbindungen der allgemeinen Formel (I) nach Anspruch 1, 2, 7, 15 oder 20
worin
R² und R⁴ ein H-Atom sind, und
R³ ein H- oder ein F-Atom ist,

32. Verbindungen der allgemeinen Formel (I) nach Anspruch 1, wie folgt
1) 6-(4-Hydroxy-phenyl)-8,9-dihydro-7H-benzocyclohepten-2-ol
2) 6-(3-Hydroxy-phenyl)-8,9-dihydro-7H-benzocyclohepten-2-ol
3) 6-Phenyl-8,9-dihydro-7H-benzocyclohepten-2-ol
4) 6-Phenyl-5-methyl-8,9-dihydro-7H-benzocyclohepten-2-ol
5) 3-Methoxy-9-methyl-8-phenyl-6,7-dihydro-5H-benzocyclohepten
6) 6-Phenyl-5-ethyl-8,9-dihydro-7H-benzocyclohepten-2-ol
7) 9-Ethyl-3-methoxy-8-phenyl-6,7-dihydro-5H-benzocyclohepten
8) 6-Phenyl-5-propyl-8,9-dihydro-7H-benzocyclohepten-2-ol
9) 3-Methoxy-8-phenyl-9-propyl-6,7-dihydro-5H-benzocyclohepten
10)6-Phenyl-5-butyl-8,9-dihydro-7H-benzocyclohepten-2-ol
11) 9-Butyl-3-methoxy-8-phenyl-6,7-dihydro-5H-benzocyclohepten
12)6-(4-Hydroxy-phenyl)-5-methyl-8,9-dihydro-7H-benzocyclohepten-2-ol
13)3-Methoxy-8-(4-methoxy-phenyl)-9-methyl-6,7-dihydro-5H-benzocyclohepten
14)4-(2-Methoxy-5-methyl-8,9-dihydro-7H-benzocyclohepten-6-yl)-phenol
15)6-(4-Methoxy-phenyl)-5-methyl-8,9-dihydro-7H-benzocyclohepten-2-ol
16)6-(3-Hydroxy-phenyl)-5-methyl-8,9-dihydro-7H-benzocyclohepten-2-ol
17)5-Ethyl-6-(4-hydroxy-phenyl)-8,9-dihydro-7H-benzocyclohepten-2-ol
18)9-Ethyl-3-methoxy-8-(4-methoxy-phenyl)-6,7-dihydro-5H-benzocyclohepten
19)4-(5-Ethyl-2-methoxy-8,9-dihydro-7H-benzocyclohepten-6-yl)-phenol
20)5-Ethyl-6-(4-methoxy-phenyl)-8,9-dihydro-7H-benzocyclohepten-2-ol
21)6-(4-Hydroxy-phenyl)-5-propyl-8,9-dihydro-7H-benzocyclohepten-2-ol
22)3-Methoxy-8-(4-methoxy-phenyl)-9-propyl-6,7-dihydro-5H-benzocyclohepten
23)4-(2-Methoxy-5-propyl-8,9-dihydro-7H-benzocyclohepten-6-yl)-phenol
24)6-(4-Methoxy-phenyl)-5-propyl-8,9-dihydro-7H-benzocyclohepten-2-ol
25)6-(4-Hydroxy-phenyl)-5-butyl-8,9-dihydro-7H-benzocyclohepten-2-ol
26)6-(3-Hydroxy-phenyl)-5-ethyl-8,9-dihydro-7H-benzocyclohepten-2-ol
27)6-(4-Hydroxy-phenyl)-5-trifluormethyl-8,9-dihydro-7H-benzocyclohepten-2-ol
28)6-(4-Hydroxy-phenyl)-5-pentafluorethyl-8,9-dihydro-7H-benzocyclohepten-2-ol
29)6-(4-Hydroxy-phenyl)-5-vinyl-8,9-dihydro-7H-benzocyclohepten-2-ol
30)3-Methoxy-8-(4-methoxy-phenyl)-9-vinyl-6,7-dihydro-5H-benzocyclohepten
31)6-(3-Hydroxy-phenyl)-5-vinyl-8,9-dihydro-7H-benzocyclohepten-2-ol
32)6-(4-Hydroxy-phenyl)-6,7,8,9-tetrahydro-5H-benzocyclohepten-2-ol
33)6-(3-Hydroxy-phenyl)-6,7,8,9-tetrahydro-5H-benzocyclohepten-2-ol
34)6-(2-Hydroxy-phenyl)-6,7,8,9-tetrahydro-5H-benzocyclohepten-2-ol
35)6-(4-Hydroxy-phenyl)-5-methyl-6,7,8,9-tetrahydro-5H-benzocyclohepten-2-ol
36)6-(4-Hydroxy-phenyl)-5-ethyl-6,7,8,9-tetrahydro-5H-benzocyclohepten-2-ol
37)6-(4-Hydroxy-phenyl)-6-methyl-6,7,8,9-tetrahydro-5H-benzocyclohepten-2-ol
38)2-Methoxy-6-(4-methoxy-phenyl)-6-methyl-6,7,8,9-tetrahydro-5H-benzocyclohepten
39)4-(2-Methoxy-6-methyl-6,7,8,9-tetrahydro-5H-benzocyclohepten-6-yl)-phenol
40)6-(4-Methoxy-phenyl)-6-methyl-6,7,8,9-tetrahydro-5H-benzocyclohepten-2-ol
41)6-(4-Hydroxy-phenyl)-6-ethyl-6,7,8,9-tetrahydro-5H-benzocyclohepten-2-ol
42)6-Ethyl-2-methoxy-6-(4-methoxy-phenyl)-6,7,8,9-tetrahydro-5H-benzocyclohepten
43)4-(6-Ethyl-2-methoxy-6,7,8,9-tetrahydro-5H-benzocyclohepten-6-yl)-phenol
44)6-Ethyl-6-(4-methoxy-phenyl)-6,7,8,9-tetrahydro-5H-benzocyclohepten-2-ol
45)6-(4-Hydroxy-phenyl)-6-vinyl-6,7,8,9-tetrahydro-5H-benzocyclohepten-2-ol
46)2-Methoxy-6-(4-methoxy-phenyl)-6-vinyl-6,7,8,9-tetrahydro-5H-benzocyclohepten
47)4-(2-Methoxy-6-vinyl-6,7,8,9-tetrahydro-5H-benzocyclohepten-6-yl)-phenol
48)6-(4-Methoxy-phenyl)-6-vinyl-6,7,8,9-tetrahydro-5H-benzocyclohepten-2-ol
49)6-(4-Hydroxy-phenyl)-4-(2-trimethylsilanyl-ethyl)-6,7,8,9-tetrahydro-5H-benzocyclohepten-2-ol
50)6-(4-Hydroxy-phenyl)-4-(2-methyldimethoxysilanyl-ethyl)-6,7,8,9-tetrahydro-5H-benzocyclohepten-2-ol
51)6-(4-Hydroxy-phenyl)-4-(2-triethoxysilanyl-ethyl)-6,7,8,9-tetrahydro-5H-benzocyclohepten-2-ol
52)6-(4-Hydroxy-phenyl)-4-vinyl-6,7,8,9-tetrahydro-5H-benzocyclohepten-2-ol
53)3-Hydroxy-8-(4-hydroxy-phenyl)-6,7,8,9-tetrahydro-5H-benzocyclohepten-1-carbaldehyd
54)3-Hydroxy-8-(4-hydroxy-phenyl)-6,7,8,9-tetrahydro-5H-benzocyclohepten-1-carbonitril
55)6-(4-Hydroxy-phenyl)-4-ethyl-6,7,8,9-tetrahydro-5H-benzocyclohepten-2-ol
56)6-(4-Hydroxy-phenyl)-4-(2-fluorethyl)-6,7,8,9-tetrahydro-5H-benzocyclohepten-2-ol
57)6-(4-Hydroxy-phenyl)-4-chlor-6,7,8,9-tetrahydro-5H-benzocyclohepten-2-ol
58)6-(4-Hydroxy-phenyl)-5-methylen-6,7,8,9-tetrahydro-5H-benzocyclohepten-2-ol
59)6-(4-Hydroxy-phenyl)-5,5-spirooxiran-6,7,8,9-tetrahydro-5H-benzocyclohepten-2-ol
60)6-(4-Hydroxy-phenyl)-5,5-spirocyclopropyl-6,7,8,9-tetrahydro-5H-benzocyclohepten-2-ol
61)5-Ethyl-6-(4-hydroxy-phenyl)-6-methyl-6,7,8,9-tetrahydro-5H-benzocyclohepten-2-ol
62)5-Ethyl-6-(4-hydroxy-phenyl)-6-ethyl-6,7,8,9-tetrahydro-5H-benzocyclohepten-2-ol
63)5-Hydroxy-2-(2-hydroxy-8,9-dihydro-7H-benzocyclohepten-6-yl)-benzonitril
64)6-(2-Chloro-4-hydroxy-phenyl)-8,9-dihydro-7H-benzocyclohepten-2-ol
65)3-Fluor-6-(4-hydroxy-phenyl)-8,9-dihydro-7H-benzocyclohepten-2-ol
66)6-(3-Fluoro-4-hydroxy-phenyl)-8,9-dihydro-7H-benzocyclohepten-2-ol
67)6-(2-Fluoro-4-hydroxy-phenyl)-8,9-dihydro-7H-benzocyclohepten-2-ol
68)3-Fluoro-4-(2-methoxy-8,9-dihydro-7H-benzocyclohepten-6-yl)-phenol
69)6-(2,3-Difluoro-4-hydroxy-phenyl)-8,9-dihydro-7H-benzocyclohepten-2-ol
70)6-(2-Chloro-3-fluoro-4-hydroxy-phenyl)-8,9-dihydro-7H-benzocyclohepten-2-ol
71)6-(2,5-Difluoro-4-hydroxy-phenyl)-8,9-dihydro-7H-benzocyclohepten-2-ol
72)6-(3,5-Difluoro-4-hydroxy-phenyl)-8,9-dihydro-7H-benzocyclohepten-2-ol
73)6-(2,6-Difluoro-4-hydroxy-phenyl)-8,9-dihydro-7H-benzocyclohepten-2-ol
74)6-(3-Chloro-4-hydroxy-phenyl)-8,9-dihydro-7H-benzocyclohepten-2-ol
75)6-(2-Methyl-4-hydroxy-phenyl)-8,9-dihydro-7H-benzocyclohepten-2-ol
76)6-(2-Ethyl-4-hydroxy-phenyl)-8,9-dihydro-7H-benzocyclohepten-2-ol
77)6-(2-Vinyl-4-hydroxy-phenyl)-8,9-dihydro-7H-benzocyclohepten-2-ol
78)6-(2-Trifluormethyl-4-hydroxy-phenyl)-8,9-dihydro-7H-benzocyclohepten-2-ol
79)6-(2-Chloro-4-hydroxy-phenyl)-5-methyl-8,9-dihydro-7H-benzocyclohepten-2-ol
80)3-Fluor-6-(4-hydroxy-phenyl)-5-methyl-8,9-dihydro-7H-benzocyclohepten-2-ol
81)6-(3-Fluoro-4-hydroxy-phenyl)-5-methyl-8,9-dihydro-7H-benzocyclohepten-2-ol
82)6-(2-Fluoro-4-hydroxy-phenyl)-5-methyl-8,9-dihydro-7H-benzocyclohepten-2-ol
83)6-(2,3-Difluoro-4-hydroxy-phenyl)-5-methyl-8,9-dihydro-7H-benzocyclohepten-2-ol
84)6-(2-Chloro-3-fluoro-4-hydroxy-phenyl)-5-methyl-8,9-dihydro-7H-benzocyclohepten-2-ol
85)6-(2,5-Difluoro-4-hydroxy-phenyl)-5-methyl-8,9-dihydro-7H-benzocyclohepten-2-ol
86)6-(3,5-Difluoro-4-hydroxy-phenyl)-5-methyl-8,9-dihydro-7H-benzocyclohepten-2-ol
87)6-(2,6-Difluoro-4-hydroxy-phenyl)-5-methyl-8,9-dihydro-7H-benzocyclohepten-2-ol
88)6-(3-Chloro-4-hydroxy-phenyl)-5-methyl-8,9-dihydro-7H-benzo-cyclohepten-2-ol
89)6-(2-Methyl-4-hydroxy-phenyl)-5-methyl-8,9-dihydro-7H-benzo-cyclohepten-2-ol
90)6-(2-Ethyl-4-hydroxy-phenyl)-5-methyl-8,9-dihydro-7H-benzocyclohepten-2-ol
91)6-(2-Vinyl-4-hydroxy-phenyl)-5-methyl-8,9-dihydro-7H-benzocyclohepten-2-ol
92)6-(2-Trifluormethyl-4-hydroxy-phenyl)-5-methyl-8,9-dihydro-7H-benzocyclohepten-2-ol
93)5-Hydroxy-2-(2-hydroxy-5-methyl-8,9-dihydro-7H-benzocyclohepten-6-yl)-benzonitril
94)6-(2-Chloro-4-hydroxy-phenyl)- 5-ethyl-8,9-dihydro-7H-benzocyclohepten-2-ol
95)5-Ethyl-3-fluor-6-(4-hydroxy-phenyl)-8,9-dihydro-7H-benzocyclohepten-2-ol
96)5-Ethyl-6-(3-Fluoro-4-hydroxy-phenyl)-8,9-dihydro-7H-benzocyclohepten-2-ol
97)6-(2-Fluoro-4-hydroxy-phenyl)- 5-ethyl-8,9-dihydro-7H-benzocyclohepten-2-ol
98)6-(2,3-Difluoro-4-hydroxy-phenyl)-5-ethyl-8,9-dihydro-7H-benzocyclohepten-2-ol
99)6-(2-Chloro-3-fluoro-4-hydroxy-phenyl)-5-ethyl-8,9-dihydro-7H-benzocyclohepten-2-ol
100) 6-(2,5-Difluoro-4-hydroxy-phenyl)-5-ethyl-8,9-dihydro-7H-benzocyclohepten-2-ol
101) 6-(3,5-Difluoro-4-hydroxy-phenyl)-5-ethyl-8,9-dihydro-7H-benzocyclohepten-2-ol
102) 6-(2,6-Difluoro-4-hydroxy-phenyl)-5-ethyl-8,9-dihydro-7H-benzocyclohepten-2-ol
103) 6-(3-Chloro-4-hydroxy-phenyl)-5-ethyl-8,9-dihydro-7H-benzocyclohepten-2-ol
104) 6-(2-Methyl-4-hydroxy-phenyl)-5-ethyl-8,9-dihydro-7H-benzocyclohepten-2-ol
105) 6-(2-Ethyl-4-hydroxy-phenyl)-5-ethyl-8,9-dihydro-7H-benzocyclohepten-2-ol
106) 6-(2-Vinyl-4-hydroxy-phenyl)-5-ethyl-8,9-dihydro-7H-benzocyclohepten-2-ol
107) 6-(2-Trifluormethyl-4-hydroxy-phenyl)-5-ethyl-8,9-dihydro-7H-benzocyclohepten-2-ol
108) 5-Hydroxy-2-(2-hydroxy-5-ethyl-8,9-dihydro-7H-benzocyclohepten-6-yl)-benzonitril
109) 5-Hydroxy-2-(2-hydroxy-5-propyl-8,9-dihydro-7H-benzocyclohepten-6-yl)-benzonitril
110) 6-(2-Chloro-4-hydroxy-phenyl)-5-propyl-8,9-dihydro-7H-benzocyclohepten-2-ol
111) 3-Fluoro-6-(4-hydroxy-phenyl)-5-propyl-8,9-dihydro-7H-benzocyclohepten-2-ol
112) 6-(3-Fluoro-4-hydroxy-phenyl)-5-propyl-8,9-dihydro-7H-benzocyclohepten-2-ol
113) 6-(2-Fluoro-4-hydroxy-phenyl)-5-propyl-8,9-dihydro-7H-benzocyclohepten-2-ol
114) 6-(2,3-Difluoro-4-hydroxy-phenyl)-5-propyl-8,9-dihydro-7H-benzocyclohepten-2-ol
115) 6-(2-Chloro-3-fluoro-4-hydroxy-phenyl)-5-propyl-8,9-dihydro-7H-benzocyclohepten-2-ol
116) 6-(2,5-Difluoro-4-hydroxy-phenyl)-5-propyl-8,9-dihydro-7H-benzocyclohepten-2-ol
117) 6-(3,5-Difluoro-4-hydroxy-phenyl)-5-propyl-8,9-dihydro-7H-benzocyclohepten-2-ol
118) 6-(2,6-Difluoro-4-hydroxy-phenyl)-5-propyl-8,9-dihydro-7H-benzocyclohepten-2-ol
119) 6-(3-Chloro-4-hydroxy-phenyl)-5-propyl-8,9-dihydro-7H-benzocyclohepten-2-ol
120) 6-(4-Hydroxy-2-methyl-phenyl)-5-propyl-8,9-dihydro-7H-benzocyclohepten-2-ol
121) 6-(2-Ethyl-4-hydroxy-phenyl)-5-propyl-8,9-dihydro-7H-benzocyclohepten-2-ol
122) 6-(4-Hydroxy-2-vinyl-phenyl)-5-propyl-8,9-dihydro-7H-benzocyclohepten-2-ol
123) 6-(4-Hydroxy-2-trifluoromethyl-phenyl)-5-propyl-8,9-dihydro-7H-benzocyclohepten-2-ol
124) 6-(2-Chloro-4-hydroxy-phenyl)-6-methyl-6,7,8,9-tetrahydro-5H-benzocyclohepten-2-ol
125) 3-Fluoro-6-(4-hydroxy-phenyl)-6-methyl-6,7,8,9-tetrahydro-5H-benzocyclohepten-2-ol
126) 6-(3-Fluoro-4-hydroxy-phenyl)-6-methyl-6,7,8,9-tetrahydro-5H-benzocyclohepten-2-ol
127) 6-(2-Fluoro-4-hydroxy-phenyl)-6-methyl-6,7,8,9-tetrahydro-5H-benzocyclohepten-2-ol
128) 6-(2,3-Difluoro-4-hydroxy-phenyl)-6-methyl-6,7,8,9-tetrahydro-5H-benzocyclohepten-2-ol
129) 6-(2-Chloro-3-fluoro-4-hydroxy-phenyl)-6-methyl-6,7,8,9-tetrahydro-5H-benzocyclohepten-2-ol
130) 6-(2,5-Difluoro-4-hydroxy-phenyl)-6-methyl-6,7,8,9-tetrahydro-5H-benzocyclohepten-2-ol
131) 6-(3,5-Difluoro-4-hydroxy-phenyl)-6-methyl-6,7,8,9-tetrahydro-5H-benzocyclohepten-2-ol
132) 6-(2,6-Difluoro-4-hydroxy-phenyl)-6-methyl-6,7,8,9-tetrahydro-5H-benzocyclohepten-2-ol
133) 6-(3-Chloro-4-hydroxy-phenyl)-6-methyl-6,7,8,9-tetrahydro-5H-benzocyclohepten-2-ol
134) 6-(2-Methyl-4-hydroxy-phenyl)-6-methyl-6,7,8,9-tetrahydro-5H-benzocyclohepten-2-ol
135) 6-(2-Ethyl-4-hydroxy-phenyl)-6-methyl-6,7,8,9-tetrahydro-5H-benzocyclohepten-2-ol
136) 6-(2-Vinyl-4-hydroxy-phenyl)-6-methyl-6,7,8,9-tetrahydro-5H-benzocyclohepten-2-ol
137) 6-(2-Trifluormethyl-4-hydroxy-phenyl)-6-methyl-6,7,8,9-tetrahydro-5H-benzocyclohepten-2-ol
138) 5-Hydroxy-2-(2-hydroxy-6-methyl-6,7,8,9-tetrahydro-5H-benzocyclohepten-6-yl)-benzonitril
139) 6-(2-Chloro-4-hydroxy-phenyl)-6-ethyl-6,7,8,9-tetrahydro-5H-benzocyclohepten-2-ol
140) 3-Fluoro-6-(4-hydroxy-phenyl)-6-ethyl-6,7,8,9-tetrahydro-5H-benzocyclohepten-2-ol
141) 6-(3-Fluoro-4-hydroxy-phenyl)-6-ethyl-6,7,8,9-tetrahydro-5H-benzocyclohepten-2-ol
142) 6-(2-Fluoro-4-hydroxy-phenyl)-6-ethyl-6,7,8,9-tetrahydro-5H-benzocyclohepten-2-ol
143) 6-(2,3-Difluoro-4-hydroxy-phenyl)-6-ethyl-6,7,8,9-tetrahydro-5H-benzocyclohepten-2-ol
144) 6-(2-Chloro-3-fluoro-4-hydroxy-phenyl)-6-ethyl-6,7,8,9-tetrahydro-5H-benzocyclohepten-2-ol
145) 6-(2,5-Difluoro-4-hydroxy-phenyl)-6-ethyl-6,7,8,9-tetrahydro-5H-benzocyclohepten-2-ol
146) 6-(3,5-Difluoro-4-hydroxy-phenyl)-6-ethyl-6,7,8,9-tetrahydro-5H-benzocyclohepten-2-ol
147) 6-(2,6-Difluoro-4-hydroxy-phenyl)-6-ethyl-6,7,8,9-tetrahydro-5H-benzocyclohepten-2-ol
148) 6-(3-Chloro-4-hydroxy-phenyl)-6-ethyl-6,7,8,9-tetrahydro-5H-benzocyclohepten-2-ol
149) 6-(2-Methyl-4-hydroxy-phenyl)-6-ethyl-6,7,8,9-tetrahydro-5H-benzocyclohepten-2-ol
150) 6-(2-Ethyl-4-hydroxy-phenyl)-6-ethyl-6,7,8,9-tetrahydro-5H-benzocyclohepten-2-ol
151) 6-(2-Vinyl-4-hydroxy-phenyl)-6-ethyl-6,7,8,9-tetrahydro-5H-benzocyclohepten-2-ol
152) 6-(2-Trifluormethyl-4-hydroxy-phenyl)-6-ethyl-6,7,8,9-tetrahydro-5H-benzocyclohepten-2-ol
153) 5-Hydroxy-2-(2-hydroxy-6-ethyl-6,7,8,9-tetrahydro-5H-benzocyclohepten-6-yl)-benzonitril
154) 6-(2-Chloro-4-hydroxy-phenyl)-6,7,8,9-tetrahydro-5H-benzocyclohepten-2-ol
155) 3-Fluoro-6-(4-hydroxy-phenyl)-6,7,8,9-tetrahydro-5H-benzocyclohepten-2-ol
156) 6-(3-Fluoro-4-hydroxy-phenyl)-6,7,8,9-tetrahydro-5H-benzocyclohepten-2-ol
157) 6-(2-Fluoro-4-hydroxy-phenyl)-6,7,8,9-tetrahydro-5H-benzocyclohepten-2-ol
158) 6-(2,3-Difluoro-4-hydroxy-phenyl)-6,7,8,9-tetrahydro-5H-benzocyclohepten-2-ol
159) 6-(2-Chloro-3-fluoro-4-hydroxy-phenyl)-6,7,8,9-tetrahydro-5H-benzocyclohepten-2-ol
160) 6-(2,5-Difluoro-4-hydroxy-phenyl)-6,7,8,9-tetrahydro-5H-benzocyclohepten-2-ol
161) 6-(3,5-Difluoro-4-hydroxy-phenyl)-6,7,8,9-tetrahydro-5H-benzocyclohepten-2-ol
162) 6-(2,6-Difluoro-4-hydroxy-phenyl)-6,7,8,9-tetrahydro-5H-benzocyclohepten-2-ol
163) 6-(3-Chloro-4-hydroxy-phenyl)-6,7,8,9-tetrahydro-5H-benzocyclohepten-2-ol
164) 6-(2-Methyl-4-hydroxy-phenyl)-6,7,8,9-tetrahydro-5H-benzocyclohepten-2-ol
165) 6-(2-Ethyl-4-hydroxy-phenyl)-6,7,8,9-tetrahydro-5H-benzocyclohepten-2-ol
166) 6-(2-Vinyl-4-hydroxy-phenyl)-6,7,8,9-tetrahydro-5H-benzocyclohepten-2-ol
167) 6-(2-Trifluormethyl-4-hydroxy-phenyl)-6,7,8,9-tetrahydro-5H-benzocyclohepten-2-ol
168) 5-Hydroxy-2-(2-hydroxy-6,7,8,9-tetrahydro-5H-benzocyclohepten-6-yl)-benzonitril
169) 5-Allyl-6-(4-hydroxy-phenyl)-8,9-dihydro-7H-benzocyclohepten-2-ol
170) 9-Allyl-3-methoxy-8-(4-methoxy-phenyl)-6,7-dihydro-5H-benzocyclohepten
171) 6-Allyl-2-methoxy-6-(4-methoxy-phenyl)-6,7,8,9-tetrahydro-benzocyclohepten-5-on
172) 6-Allyl-2-methoxy-6-(4-methoxy-phenyl)-6,7,8,9-tetrahydro-5H-benzocyclohepten
173) 6-Allyl-6-(4-hydroxy-phenyl)-6,7,8,9-tetrahydro-5H-benzocyclohepten-2-ol
174) 2-Methoxy-6-(4-methoxy-phenyl)-6,7,8,9-tetrahydro-benzocyclohepten-5-on
175) 2-Methoxy-6-(4-methoxy-phenyl)-6-propyl-6,7,8,9-tetrahydro-benzocyclohepten-5-on
176) 2-Methoxy-6-(4-methoxy-phenyl)-6-propyl-6,7,8,9-tetrahydro-5H-benzocyclohepten
177) 6-(4-Hydroxy-phenyl)-6-propyl-6,7,8,9-tetrahydro-5H-benzocyclohepten-2-ol
178) 5-Allyl-6-(4-hydroxy-phenyl)-6-methyl-6,7,8,9-tetrahydro-5H-benzocyclohepten-2-ol
179) 5-Allyl-6-(4-hydroxy-phenyl)-6-ethyl-6,7,8,9-tetrahydro-5H-benzocyclohepten-2-ol
180) 6-(4-Hydroxy-phenyl)-4-trifluoromethyl-6,7,8,9-tetrahydro-5H-benzocyclohepten-2-ol
181) 4,6-Diethyl-6-(4-Hydroxy-phenyl)-6,7,8,9-tetrahydro-5H-benzocyclohepten-2-ol
182) 4-Ethyl-6-(4-Hydroxy-phenyl)-6-methyl-6,7,8,9-tetrahydro-5H-benzocyclohepten-2-ol
183) 6-(3-Hydroxy-phenyl)-6-methyl-6,7,8,9-tetrahydro-5H-benzocyclohepten-2-ol
184) 6-(3-Hydroxy-phenyl)-5-butyl-6,7,8,9-tetrahydro-5H-benzocyclohepten-2-ol
185) 6-(4-Hydroxy-phenyl)-5-propyl-6,7,8,9-tetrahydro-5H-benzocyclohepten-2-ol
186) 6-(3-Hydroxy-phenyl)-5-propyl-6,7,8,9-tetrahydro-5H-benzocyclohepten-2-ol
187) 6-(4-Hydroxy-phenyl)-5-propenyl-8,9-dihydro-7H-benzocyclohepten-2-ol
188) 6-(4-Hydroxy-phenyl)-5,5-dimethyl-8,9-dihydro-5H-benzocyclohepten-2-ol
189) 6-(4-Hydroxy-phenyl)-5,5-dimethyl-6,7,8,9-tetrahydro-5H-benzocyclohepten-2-ol
190) 5-Ethyl-6-(4-hydroxy-phenyl)-5-methyl-8,9-dihydro-5H-benzocyclohepten-2-ol
191) 5,5-Diethyl-6-(4-hydroxy-phenyl)-8,9-dihydro-5H-benzocyclohepten-2-ol
192) 6-(4-Hydroxyphenyl)-5-phenyl-8,9-dihydro-7H-benzocyclohepten-2-ol
193) 6-(4-Hydroxyphenyl)-5-(3-methylthiophen-2-yl)-8,9-dihydro-7H-benzocyclohepten-2-ol

33. Pharmazeutische Zusammensetzungen, die mindestens eine Verbindung der allgemeinen Formel I nach einem der Ansprüche 1 bis 32, gegebenenfalls zusammen mit pharmazeutisch verträglichen Hilfs und Trägerstoffen enthalten.

34. Verbindungen der allgemeinen Formel I nach einem der Ansprüche 1 bis 32 zur Verwendung als Medikament.
